# EUROPÄISCHE PATENTSCHRIFT

(11) **EP 3 511 482 B1**
(45) Veröffentlichungstag und Bekanntmachung des Hinweises auf die Patenterteilung: **09.02.2022**
(21) Anmeldenummer: 18210985.0
(22) Anmeldetag: 07.12.2018
(51) Int. Cl.: E04F 15/02, G08B 13/10, H02J 50/10, A61B 5/11, F21V 33/00, G08B 21/04, G07C 9/00

(54) **BELAGSELEMENT UND SYSTEM MIT WENIGSTENS EINEM BELAGSELEMENT**
COVERING ELEMENT AND SYSTEM WITH AT LEAST ONE COVERING ELEMENT
ÉLÉMENT DE REVÊTEMENT ET SYSTÈME COMPRENANT AU MOINS UN ÉLÉMENT DE REVÊTEMENT

(30) Priorität: 15.01.2018 DE 102018000288; 02.03.2018 DE 102018001649
(43) Veröffentlichungstag der Anmeldung: 17.07.2019
(73) Patentinhaber: Parador GmbH, 48653 Coesfeld (DE)
(72) Erfinder: Petersen, Frank, 48653 Coesfeld (DE); Verstegge, Christian, 48599 Gronau-Epe (DE); Damiecki, David, 48653 Coesfeld (DE); Wolf, Matthias, 48653 Coesfeld (DE); Paschert, Martin, 48703 Stadtlohn (DE)
(74) Vertreter: Von Rohr Patentanwälte Partnerschaft mbB

(56) Entgegenhaltungen:
- EP-A1- 2 927 018
- WO-A1-2016/118796
- DE-A1-102012 214 379
- DE-A1-102016 010 583
- DE-U1-202015 007 999
- US-A1- 2014 133 137

## Beschreibung

Die Erfindung betrifft ein Belagselement, vorzugsweise für einen eine Mehrzahl von Belagselementen und/oder weiteren Belagselementen aufweisenden Belag zur Verwendung als Fußboden-, Wand- und/oder Deckenbelag, mit wenigstens einem Grundkörper und wenigstens einer Funktionsschicht. Des Weiteren betrifft die Erfindung ein System mit wenigstens einem Belagselement, insbesondere einem Belagselement der vorgenannten Art, wobei das System eine Verarbeitungseinrichtung und wenigstens ein weiteres Belagselement aufweist.

Bei den bekannten Belagselementen, beispielsweise zur Boden- oder Deckenverkleidung, sind die Ausgestaltungsmöglichkeiten in der Regel auf die Erzielung eines bestimmten optischen Eindrucks und auf die Gebrauchstauglichkeit im Alltag beschränkt. Funktionale Elemente jeglicher Art müssen jeweils in situationsangepasster Weise installiert werden. Dies erfordert neben einer sorgfältigen Planung in der Regel auch eine Festlegung auf gewünschte Funktionen im Vorfeld des Verbaus von Belagselementen der vorgenannten Art.

Zudem sind bei einem Bauprozess für ein Gebäude in der Regel mehrere Gewerke notwendig, um eine Wand oder einen Boden des Baukörpers vollständig auszurüsten. Funktionale Elemente wie Leuchten, Anschlüsse und die entsprechenden Zuleitungen müssen installiert werden. Die Oberfläche wird daraufhin in der gewünschten Weise, insbesondere mit Belagselementen wie Paneelelementen, verkleidet. Eine solch aufwendige Installation ist häufig nur bei einem Neubau wirtschaftlich sinnvoll, während eine nachträgliche Installation mit hohen Kosten verbunden ist, sofern sie überhaupt möglich ist.

Belagselemente der vorgenannten Art mit einer elektrischen Funktionalität, die durch die Funktionsschicht zur Verfügung gestellt wird, lassen sich mit Smart-Home-Anwendungen koppeln, die zunehmend Verbreitung finden. Hierzu zählen beispielsweise die Automatisierung von Gebäudefunktionen und die Verflechtung elektronischer Komponenten mit dem Wohnraum, insbesondere unter Verwendung eines Bussystems. Es besteht jedoch in diesem Zusammenhang die Problematik, dass Systeme für Smart-Home-Anwendungen auf unterschiedlichen Standards basieren. Zusätzlich zur Festlegung der räumlichen Anordnung funktionaler Elemente ist bei einem Bauprozess auch die Entscheidung für einen solchen Standard in der Regel bereits im Vorfeld notwendig.

Die elektrische Funktionalität bzw. die Funktionselemente können bei einem Belagselement in der elektrischen Funktionsschicht integriert sein. Hierbei kann die elektrische Funktionsschicht dem Belagselement zugeordnet und/oder in dieses integriert sein. Die Funktionsschicht kann ferner eine Detektierfunktion bzw. Sensoren zur Erfassung von Personen und/oder ein Leuchtmittel aufweisen. Bei einem Belag mit Belagselementen der vorgenannten Art ist die elektrische Funktionalität für den gesamten Belag vorgesehen. Beispielsweise können einzelne Belagselemente elektrisch kontaktierend miteinander verbunden werden. Gleichfalls ist es auch denkbar, die Funktionsschicht separat von den Belagselementen, insbesondere ober- und/oder unterhalb der Belagselemente, anzuordnen.

Nachteilig ist hierbei, dass, sofern eine elektrische Funktionalität für einen Belag gewünscht ist, die gesamte Fläche des Belages die elektrische Funktionalität in Form einer Funktionsschicht aufweist. Demzufolge kann ein solcher Belag mit hohen Kosten verbunden sein.

Die DE 20 2015 007 999 U1 betrifft einen Plattenkörper für einen Boden-, Wand- oder Deckenbelag, wobei zwischen einer Trägerplatte und einer Deckschicht wenigstens eine eine gedruckte Schaltung aufweisende Funktionsschicht vorgesehen ist.

Die US 2014/0133137 A1 betrifft ein modulares Element, dass wenigstens ein Leuchtelement aufweist.

Die DE 10 2012 214 379 A1 betrifft ein Verfahren zur Herstellung eines Untergrundbelags, wobei eine erste Schicht bereitgestellt wird, wobei eine Sensorschicht auf die erste Schicht aufgebracht und wobei eine zweite Schicht auf die Sensorschicht aufgebracht wird.

Die WO 2016/118796 A1 betrifft ein Bodenbelagssystem, in dem Sensoren integriert sind.

Die DE 10 2016 010 583 A1 betrifft eine Folie zur Beschichtung von Boden-, Wand- und/oder Deckenbelägen und/oder von Möbelbauteilen, mit wenigstens einer Trägerschicht, wenigstens einer Dekorschicht und/oder wenigstens einer oberseitigen Deckschicht und mit einer unterseitigen Haftschicht. Es ist vorgesehen, dass zwischen der Haftschicht und der Deckschicht zumindest bereichsweise wenigstens eine Funktionsschicht mit einer gedruckten Schaltung vorgesehen ist.

Vor diesem Hintergrund ist es die Aufgabe der vorliegenden Erfindung, die vorgenannten Nachteile im Stand der Technik zu vermeiden oder aber zumindest im Wesentlichen zu reduzieren. Insbesondere ist es die Aufgabe der vorliegenden Erfindung, die Flexibilität beim Einsatz funktionaler Elemente im Zusammenhang mit Belagselementen der eingangs genannten Art zu erhöhen und den Anwendungsbereich von Belagselementen entsprechend zu vergrößern. Des Weiteren ist es insbesondere die Aufgabe der vorliegenden Erfindung, die Bereitstellung einer elektrischen Funktionalität für einen Belag kostengünstig zu ermöglichen, wobei die Installation der Belagselemente vorzugsweise kabelfrei erfolgt.

Die vorgenannte Aufgabe wird erfindungsgemäß zumindest im Wesentlichen durch den Anspruch 1 gelöst.

Erfindungsgemäß ist vorgesehen, dass die Funktionsschicht eine Übertragungseinrichtung zur Übertragung von Informationen und eine Messeinrichtung zur Erfassung von Informationen aufweist, wobei die Messeinrichtung derart mit der Übertragungseinrichtung gekoppelt ist, dass die Übertragungseinrichtung zur kabellosen Übertragung der von der Messeinrichtung empfangenen Informationen ausgebildet ist und wobei das Belagselement an allen Seitenrändern umlaufend elektrisch kontaktierungslos und elektrisch kontaktfrei ausgebildet ist.

Die in den Schichtaufbau eines solchen Belagselementes integrierte Funktionsschicht erlaubt einen flexiblen Einsatz von funktionalen Elementen im Zusammenhang mit dem Belagselement. Die Funktionalität hängt dabei letztlich von der Beschaffenheit bzw. Ausgestaltung der Funktionsschicht ab. Beispielsweise kann die Funktionsschicht wenigstens einen Aktor in Form einer Leuchtschicht aufweisen und/oder als gedruckte Schaltung ausgebildet sein.

Die Integration einer Funktionalität in Form einer sogenannten Printed Intelligence in Belagselemente führt zu einem hohen Maß an Modularität. Neben einer gedruckten Funktionsschicht sind auch Funktionsschichten denkbar, bei denen die elektronischen Bestandteile bzw. Bauteilen auf einen Träger, insbesondere eine Folie, montiert und/oder angebracht sind. Insbesondere kann die Funktionsschicht über die sogenannte Chip-on-Board-Technologie aufgebracht sein, bei der ungehäuste Halbleiter-Chips in Direktmontage elektrisch kontaktierend mit der Leiterplatte verbunden sind.

Letztlich können durch die Funktionsschicht unterschiedliche elektrische Funktionalitäten bereitgestellt werden. Dadurch ergeben sich Vorteile insbesondere gegenüber der Anordnung funktionaler Elemente hinter einer Wand- oder Bodenverkleidung. Bei der Montage der Belagselemente, das heißt insbesondere bei der Installation eines Decken-, Wand- und/oder Bodenbelags, können die Belagselemente mit verschiedenen Funktionalitäten in gewünschter und nahezu beliebiger Weise miteinander kombiniert werden. Erfindungsgemäß ist dabei vorgesehen, dass die einzelnen Belagselemente nicht miteinander gekoppelt sind und so gesehen "autark" Informationen erfassen und verarbeiten können.

Bei der Veränderung der Anwendungssituation lassen sich funktionale Elemente, die durch in dem Belagselement integrierte Aktoren gebildet sein können, leicht anpassen. Dies kann im einfachsten Fall durch einen simplen Austausch des Belagselementes gegen ein neues Belagselement mit einer entsprechend anders gestalteten Funktionalität erfolgen. Im Falle einer Boden-, Wand- und/oder Deckenverkleidung wird eine Anpassung der funktionalen Elemente dementsprechend erleichtert, da die funktionalen Elemente als Aktoren in der Funktionsschicht des von außen zugänglichen Boden-, Wand- bzw. Deckenbelags vorgesehen sein können. Ein Austausch dieser Elemente ist demnach mit deutlich geringerem Aufwand verbunden, als ein Austausch von Elementen hinter einer solchen Verkleidung.

Durch die Integration funktionaler Einheiten in das Belagselement wird die gewünschte Funktionalität bereitgestellt und gleichzeitig ein dezentes und ansprechendes, optisches Erscheinungsbild einer mit den erfindungsgemäßen Belagselementen verkleideten Oberfläche erreicht. Dies gilt insbesondere für den inaktiven Zustand der funktionalen Einheiten. Es ist beispielsweise möglich, dass eine Leuchtschicht im Belagselement zu Beleuchtungszwecken eingesetzt wird, jedoch kein Leuchtmittel sichtbar ist, wenn auf eine künstliche Beleuchtung verzichtet werden kann oder soll.

Erfindungsgemäß zeichnet sich das Belagselement insbesondere dadurch aus, dass es an weiteren Belagselementen, die keine elektrische Funktionalität und/oder Funktionsschicht aufweisen, angeordnet bzw. mit diesen gekoppelt werden kann. So ist es beispielsweise möglich, einen Belag mit einer elektrischen Funktionalität bereitzustellen, wobei die elektrische Funktionalität lediglich in wenigen Belagselementen des Belages integriert sein kann. Dies reduziert die Kosten der Bereitstellung einer elektrischen Funktionalität im Bereich eines Wand-, Boden- oder Deckenbelages, da letztlich einzelne erfindungsgemäße Belagselemente mit weiteren, nicht erfindungsgemäßen Belagselementen kombiniert werden können. Auch die nachträgliche Bereitstellung einer elektrischen Funktionalität innerhalb eines bereits vorhandenen Wand-, Boden- oder Deckenbelages kann sehr einfach erfolgen, da hierfür lediglich einzelne weitere Belagselemente gegen erfindungsgemäße Belagselemente ausgetauscht werden können.

Derartige erfindungsgemäße Belagselemente können beispielsweise im Bereich der Sicherheitstechnik zur Erfassung der Anwesenheit von Personen und/oder zur Steuerung bzw. Regelung von Menschenmengen genutzt bzw. verwendet werden. Ein Einsatz bzw. eine Verwendung in der Gesundheitsbranche, beispielsweise zur schnellen Erkennung eines Sturzes von Patienten und/oder einer hilfsbedürftigen Person, ist ebenfalls denkbar.

Im Zusammenhang mit der vorliegenden Erfindung sind unter elektrisch kontaktierunglosen und/oder elektrisch kontaktfreien Seitenrändern solche Seitenränder zu verstehen, die beispielsweise elektrisch isolierend ausgebildet sein können. Letztlich bezieht sich das Merkmal darauf, dass die Seitenränder signalisolierend und/oder informationsaustauch-isolierend ausgebildet sind. Sofern das Belagselement beispielsweise in einem Belag mit Belagselementen und weiteren Belagselementen integriert wird, so weist das Belagselement keinen elektrischen Kontakt zum Informationsaustausch mit benachbarten Belagselementen und/oder weiteren Belagselementen auf. Die Funktionsschicht kann an dem Grundkörper angeordnet und/oder mit diesem verbunden sein.

Der durch wenigstens ein Belagselement gebildete Belag kann beispielsweise als Vinyl-Belag, als modularer Boden, Laminat und/oder Parkett ausgebildet sein.

Insbesondere ist unter einer Funktionsschicht im Sinne der vorliegenden Erfindung eine elektrisch leitende Funktionsschicht zu verstehen, insbesondere eine Leiterplatte, die elektronische Bauteile, insbesondere montiert und/oder gedruckt auf der Leiterplatte, aufweist. Dabei versteht es sich letztlich, dass die Funktionsschicht und/oder die Funktionsschichten auf einer Trägerschicht aufgebracht und/oder an einer Trägerschicht angeordnet sein können. Demgemäß kann sich die Funktionsschicht bereichsweise auf dem Belagselement erstrecken, wobei die die Funktionsschicht und/oder die Funktionsschichten aufweisende Trägerschicht sich über die gesamte Breite und/oder Länge des Belagselementes erstrecken kann. Die Leiterplatte bzw. die Funktionsschicht kann auf die Trägerschicht aufgedruckt sein, wobei unterschiedliche Druckmethoden denkbar sind: Siebdruck, Tampondruck, Digitaldruck und/oder Bedampfung. Des Weiteren ist auch eine getrennte Herstellung der Funktionsschicht bzw. der Leiterplatte möglich, wobei die Funktionsschicht vor Anordnung auf dem Belagselement mit der Trägerschicht verbunden werden kann.

Erfindungsgemäß ist vorgesehen, dass die Funktionsschicht, insbesondere die Leiterplatte, zu wenigstens zwei Seitenrändern beabstandet ist, insbesondere wobei der Abstand mindestens 0,1 % der Breite und/oder der Länge des Belagselementes ist, bevorzugt zwischen 0,1 % bis 40 %, weiter bevorzugt zwischen 1 % bis 20 %, liegt. Die Beabstandung der Funktionsschicht zu wenigstens zwei Seitenrändern, vorzugsweise allen Seitenrändern, kann die elektrische Isolation an den Seitenrändern gewährleisten. Vorteilhaft ist hierbei, dass die Funktionsschicht an ihren an den Seitenrändern des Belagselementes grenzenden Rändern nicht zusätzlich elektrisch isoliert werden muss. Darüber hinaus kann ein Zuschnitt des Belagselementes, insbesondere an den Randseiten des Belages, - zumindest in gewissen Bereichen - ohne eine Beschädigung der Funktionsschicht erfolgen.

Sofern das Belagselement im Bereich der Sicherheitstechnik, insbesondere gekoppelt mit einer Alarmeinrichtung, eingesetzt bzw. verwendet wird, so ist es vorteilhaft, dass auch randseitige Belagselemente eines Belages die elektrische Funktionalität aufweisen können, so dass sowohl zugeschnittene als auch nicht zugeschnittene Belagselemente eine elektrische Funktionalität aufweisen können. Insbesondere kann die elektrische Funktionalität in beliebigen Bereichen des Belages gewährleistet werden. Durch die Beabstandung der Funktionsschicht zu den Seitenrändern kann eine Beschädigung der Funktionsschicht beim Zuschnitt des Belagselements sicher verhindert werden.

Darüber hinaus ist bei einer bevorzugten Ausführungsform des Belagselementes die Funktionsschicht mittig auf den Grundkörper angeordnet und/oder die Funktionsschicht erstreckt sich zwischen 1 % bis 85 %, bevorzugt zwischen 5 % und 75 %, weiter bevorzugt zwischen 10 % bis 50 % und insbesondere zumindest im Wesentlichen 20 %, der Breite und/oder der Länge des Belagselementes auf dem Grundkörper. Sowohl die mittige Anordnung als auch eine geringere Breite und/oder Länge der Funktionsschicht im Verhältnis zum Grundkörper kann die zuvor genannten Vorteile einer Beabstandung der Funktionsschicht von den Seitenrändern gewährleisten. So kann durch die mittige Anordnung der Funktionsschicht auf dem Grundkörper ein Zuschnitt bzw. eine Anpassung des Belagselementes an einen, insbesondere bereits verlegten, Belag erfolgen.

Des Weiteren kann eine Mehrzahl von Funktionsschichten, bevorzugt mehr als zwei, weiter bevorzugt zwischen 2 bis 15, weiter bevorzugt zwischen 2 bis 5 und insbesondere zumindest im Wesentlichen 4, vorgesehen sein. Die Funktionsschichten können unabhängig voneinander ausgebildet sein, das heißt nicht miteinander gekoppelt sein. Insbesondere können die Funktionsschichten sich voneinander unterscheidende elektrische Funktionseinheiten aufweisen. So kann eine Funktionsschicht als Leuchtschicht ausgebildet sein und eine weitere Funktionsschicht kann einen mechanischen Aktor zur Erfassung von Bewegungen aufweisen. Grundsätzlich ist es auch möglich, dass die Funktionsschichten zumindest im Wesentlichen dieselbe Funktionalität aufweisen, so dass bei Zuschnitt des Belagselementes und einer damit verbundenen Abtrennung von wenigstens einer Funktionsschicht die elektrische Funktionalität des Belagselementes weiterhin gewährleistet ist. Durch die Bereitstellung einer Mehrzahl von Funktionsschichten kann bei Anpassung des Belagselementes eine Beschädigung der Funktionsschicht zumindest im Wesentlichen vermieden werden.

In diesem Zusammenhang sind die Funktionsschichten vorzugsweise voneinander beabstandet angeordnet, und zwar bevorzugt in einer Funktionsschichtebene. So kann eine Abtrennung bzw. ein Schnitt an dem Belagselement in dem sich zwischen den Funktionsschichten ergebenden Freiraum erfolgen.

Zudem kann die Übertragungseinrichtung zur Verarbeitung der von der Messeinrichtung übertragenen Informationen ausgebildet sein. So können beispielsweise neben den durch die Messeinrichtung gemessenen Informationen auch weitere Informationen übertragen werden, die auf einer Analyse bzw. Verarbeitung oder Berechnung der gemessenen Informationen beruhen können. So kann in der Übertragungseinrichtung ermittelt werden, ob eine Person sich oberhalb des Belagselementes und/oder in der Nähe zu dem Belagselement befindet.

Des Weiteren kann die Übertragungseinrichtung wenigstens eine Antenne zum Senden und/oder Empfangen von Informationen, die insbesondere über elektromagnetische Wellen übertragbar sind, aufweisen. Die Ansteuerung der Antenne kann beispielsweise in Abhängigkeit der von der Messeinrichtung erfassten Informationen erfolgen, insbesondere indem die Informationen in der Übertragungseinrichtung verarbeitet werden. Durch die Antenne kann eine kabellose Übertragung der Informationen gewährleistet werden. Vorteilhaft ist hierbei, dass eine aufwendige Verkabelung des Belagselementes vermieden werden kann. Die Antenne kann derart ausgebildet sein, dass sie in verschiedenen Frequenzbereichen die Daten übertragen kann, wobei der Frequenzbereich in Abhängigkeit des Verwendungszwecks des Belagselementes ausgewählt sein kann.

Alternativ oder zusätzlich kann die Übertragungseinrichtung zur Codierung und/oder Verschlüsselung der Informationen ausgebildet sein. Insbesondere im Zusammenhang mit einer gewünschten Datensicherheit ist eine Verschlüsselung der durch die Messeinrichtung erfassten Informationen sinnvoll, da so gewährleistet werden kann, dass der gewünschte und vorgegebene Empfänger die Informationen erhält und diese verarbeiten kann, ohne dass weitere, mögliche Empfänger die Informationen verarbeiten oder auslesen können.

Bei einer weiteren, besonders bevorzugten Ausgestaltung des Erfindungsgedankens weist die Messeinrichtung wenigstens einen Sensor, vorzugsweise zur Erfassung einer physikalischen Größe, auf. Der Sensor kann zur Messung von Druck, Feuchtigkeit, Beleuchtungsstärke, Temperatur, Schwingungen und/oder Bewegungen ausgebildet sein. Alternativ oder zusätzlich ist der Sensor als Lichtsensor, Feuchtigkeitssensor, Näherungssensor, Wärmesensor und/oder Bewegungssensor, insbesondere Druck- und/oder Schwingungssensor, ausgebildet. Die vorgenannten Sensoren ermöglichen die elektrische Funktionalität der Funktionsschicht. Beispielsweise kann beim Einsatz im Bereich der Sicherheitstechnik eine Veränderung der Temperatur und der Druckbelastung nahe des Belagselements gezielt erfasst werden. So kann das Belagselement den Eintritt einer Person in einen Raum registrieren, sei es durch die durch die Person abgestrahlte Wärme, durch das Gewicht der Person, das auf dem Belagselement lastet, und/oder durch die bei der Bewegung der Person erzeugten Schwingungen im Boden.

Das Signal des Sensors kann dazu genutzt werden, Stellglieder eines Regelkreises derart zu steuern, dass ein Sollwert für eine bestimmte physikalische Größe erreicht wird. Dies betrifft beispielsweise die Temperatur oder die Beleuchtungsstärke. Die Stellglieder eines Regelkreises, die aufgrund eines Sensorsignals aktiviert bzw. angesteuert werden, können sowohl außerhalb des Belagselementes vorgesehen sein als auch in Form von Aktoren in der Funktionsschicht des Belagselementes vorliegen.

Die Messeinrichtung kann einen Drucksensor aufweisen, welcher insbesondere rasterförmig und/oder als Raster ausgebildet in der Funktionsschicht integriert ist. Darüber hinaus kann ebenfalls wenigstens ein Rasterpunkt des Drucksensors die Schwingung der gesamten Trägerplatte aufnehmen und, vorzugsweise, dadurch einen Impuls auslösen. Der Drucksensor beruht darauf, dass die Druckbelastung auf dem Belagselement einen Impuls erzeugt. Der Drucksensor kann beispielsweise als piezoelektrischer Drucksensor ausgebildet sein, bei dem mittels Druck durch Ladungstrennung eine elektrische Spannung in einem Kristall erzeugt wird. Die Ladung kann durch einen Ladungsverstärker in eine proportionale elektrische Spannung umgeformt werden, wobei der piezoelektrische Sensor auf einer Korrelation zwischen Kraft und Druck beruht und die Kraft misst. Vorteilhaft an einem piezoelektrischen Sensor ist, dass durch die Druckbelastung eine Spannung erzeugt und insbesondere keine äußere Spannungsversorgung bzw. Energieversorgung benötigt wird. So kann sich die Messeinrichtung bei Erfassung einer Druckbelastung selbst mit Energie versorgen und diese Energie insbesondere auch an die Übertragungseinrichtung weitergeben.

Vorzugsweise ist die Messeinrichtung zum Auslesen eines Zugangskontrollmittels, insbesondere einer RFID-Karte, und/oder zum Empfangen eines Taktsignals, von Energie und/oder von Personenauthentifizierungsdaten eines Zugangskontrollmittels ausgebildet. Über das Zugangskontrollmittel kann ein möglicher Zugriff zu einem Raum gesteuert werden, wobei die Messeinrichtung und damit einhergehend das Belagselement das Zugangskontrollmittel auslesen und damit sicherstellen kann, dass nur autorisierte Personen einen bestimmten Bereich bzw. Raum betreten können. Des Weiteren kann zusätzlich oder alternativ von dem Zugangskontrollmittel auch Energie, insbesondere zur Energieversorgung der Funktionsschicht, an die Messeinrichtung übertragen werden. Das Zugangskontrollmittel kann derart mit der Messeinrichtung gekoppelt sein, dass die Messeinrichtung Personenauthentifizierungsdaten von dem Zugangskontrollmittel empfangen kann. Beispielsweise ist als Zugangskontrollmittel eine RFID-Karte vorgesehen. RFID (radiofrequency identification / Identifizierung mit Hilfe elektromagnetischer Wellen) kennzeichnet ein automatisches und berührungsloses Identifizieren sowie Lokalisieren von Objekten sowie Lebewesen mittels Radiowellen und stellt somit eine Technologie für ein Sender-Empfänger-System dar. In diesem Fall wäre der Sender das Zugangskontrollmittel und der Empfänger die Messeinrichtung.

Vorteilhafterweise wird ein aktives Handeln von der zu authentifizierenden Person vermieden, so dass die Person nicht mehr, wie bisher üblich, das Zugangskontrollmittel direkt vor einem Lesegerät positionieren muss. Das Zugangskontrollmittel kann ferner auch einem Objekt oder einem Lebewesen implantiert werden, so dass automatisch bei Betreten des Belagselementes eine Authentifizierung erfolgen kann. Insbesondere lässt sich durch die Kopplung des Zugangskontrollmittels mit der Messeinrichtung ein gesicherter sowie beschränkter Zugang zu einem Raum bzw. einem abgeschlossenen Bereich ermöglichen. Neben oder zusätzlich zu einer Zugangsberechtigung kann eine Datenerfassung durch das Zugangskontrollmittel derart erfolgen, dass die Personen durch die von der Messeinrichtung erfassten Informationen authentifizierbar bzw. zuordnenbar sind.

Alternativ oder zusätzlich kann ein Lesegerät zum Auslesen eines Zugangskontrollmittels vorgesehen sein, wobei das Lesegerät separat von dem Belagselement vorgesehen ist, jedoch dem Belagselement zugeordnet sein kann. Als Zugangskontrollmittel kann eine RFID-Karte dienen. Das Lesegerät kann Informationen, vorzugsweise des Zugangskontrollmittels, mit dem Belagselement austauschen und/oder an das Belagselement übertragen und/oder von dem Belagselement empfangen. Insbesondere kann das Belagselement zur Erfassung der Anwesenheit und/oder zum Tracking und/oder zur Ortung des Zugangskontrollmittels ausgebildet sein.

Vorzugsweise kann eine Identifizierung bzw. Authentifizierung der Person durch Auslesen des Zugangskontrollmittels mit dem Lesegerät erfolgen, wobei nach erfolgter Identifizierung bzw. Authentifizierung ein Bewegungsprofil der Person durch den Belag bzw. durch die Belagselemente erstellbar ist.

Besonders bevorzugt ist die Messeinrichtung zur Digitalwandlung gemessener, analoger Informationen in digitale Informationen und/oder zur Übertragung digitaler Informationen an die Übertragungseinrichtung ausgebildet. Zudem kann die Digitalwandlung alternativ oder zusätzlich in der Übertragungseinrichtung erfolgen. Die Umwandlung der gemessenen, analogen Informationen in digitale Informationen bietet den Vorteil, dass diese mittels elektromagnetischer Wellen durch die Antenne der Übertragungseinrichtung übertragbar sind.

Darüber hinaus kann bei einer weiteren bevorzugten Ausführungsform des Erfindungsgedankens vorgesehen sein, dass die Funktionsschicht eine Speichereinrichtung zur Speicherung der durch die Übertragungseinrichtung verarbeiteten und/oder empfangenen und/oder durch die Messeinrichtung erfassten Informationen aufweist. Die Speichereinrichtung kann mit der Messeinrichtung und/oder der Übertragungseinrichtung gekoppelt sein. Beispielsweise werden die in der Speichereinrichtung gespeicherten Informationen zur Verarbeitung der durch die Messeinrichtung empfangenen Informationen in der Übertragungseinrichtung genutzt, insbesondere zum Vergleich. In der Speichereinrichtung können im Zusammenhang mit einem Zugangskontrollmittel die Personenauthentifizierungsdaten sowie der Zeitpunkt der Erfassung gespeichert werden, so dass rückwirkend auf die Anwesenheit von Personen in einem Raum - durch das Sender-Empfänger-Verhältnis zwischen dem Zugangskontrollmittel und der Messeinrichtung - geschlossen werden kann.

Des Weiteren kann der Funktionsschicht eine Verarbeitungseinrichtung zugeordnet sein, wobei die Verarbeitungseinrichtung zum Empfangen von durch die Übertragungseinrichtung übertragenen Informationen vorgesehen ist. Die Verarbeitungseinrichtung kann als externer Controller ausgebildet sein. Sie kann ferner als Bestandteil bzw. zur Integration an eine Smart-Home-Anwendung vorgesehen sein und/oder eine Kompatibilität für ein Bussystem aufweisen. Insbesondere ist das Belagselement durch die Verarbeitungseinrichtung regel- und/oder steuerbar, vorzugsweise in Abhängigkeit der durch die Messeinrichtung erfassten Informationen. Darüber hinaus kann die Verarbeitungseinrichtung in Abhängigkeit der durch die Messeinrichtung, insbesondere dem Sensor, erfassten Informationen, vorzugsweise einer physikalischen Größe, Steuerabläufe bei Über- oder Unterschreiten eines vorgebbaren Sollwertes initiieren.

Weiterhin kann das Belagselement und/oder die Funktionsschicht eine Anzeigeeinrichtung aufweisen, wobei die Anzeigeeinrichtung zur akustischen, taktilen, haptischen, olfaktometrischen und/oder visuellen Vermittlung von Informationen ausgebildet ist, insbesondere wobei die Anzeigeeinrichtung oberseitig auf dem Grundkörper und/oder der Funktionsschicht angeordnet und/oder als Leuchtschicht ausgebildet ist. Vorzugsweise ist die Anzeigeeinrichtung als LC-Display und/oder als Matrixdisplay, insbesondere als elektronisches Papier, ausgebildet und/oder weist bevorzugt Leiterbahnen zur Energieübertragung in Form von Strom auf. Die Anzeigeeinrichtung dient zur Informationsvermittlung bzw. Informierung einer Person, die sich räumlich in der Nähe zu dem erfindungsgemäßen Belagselement befindet. Die Person kann optisch und/oder akustisch informiert werden, wobei bevorzugt eine visuelle Vermittlung genutzt wird. Beispielsweise kann die Anzeigeeinrichtung das Gewicht der Person, welches durch die Messeinrichtung gemessen werden kann, der Person anzeigen. Aber auch jegliche weiteren Informationen, die entweder die Person oder den Raum betreffen, können der Person angezeigt werden.

Zudem kann bei einer weiteren bevorzugten Ausgestaltung der Erfindung der Funktionsschicht wenigstens ein Energieversorgungsmittel zugeordnet und/oder in der Funktionsschicht integriert sein. Das Energieversorgungsmittel kann einen, vorzugsweise gedruckten, Energiespeicher, insbesondere eine Dünnschichtbatterie, aufweisen. Alternativ oder zusätzlich kann das Energieversorgungsmittel zum, vorzugsweise kabellosen, Empfangen von Energie durch die Messeinrichtung ausgebildet sein. Folglich lässt sich eine energieautarke Versorgung des Belagselementes gewährleisten. Beispielsweise weist die Messeinrichtung wenigstens ein PiezoElement auf, bei dem aufgrund der Druckbelastung eine elektrische Spannung erzeugt werden kann, die letztlich zur Energieversorgung der Funktionsschicht über das Energieversorgungsmittel dienen kann. Des Weiteren kann das Energieversorgungsmittel auch ein photovoltaisches Element aufweisen, wobei Lichtenergie in elektrische Energie mittels Solarzellen umgewandelt werden kann. Außerdem ist es auch denkbar, das Energieversorgungsmittel über Induktion zu laden. In diesem Zusammenhang versteht es sich, dass die für die Funktionsschicht benötigte Energie in Form von Strom bzw. in Form von elektrischer Energie zur Verfügung gestellt werden kann.

Insbesondere bei einer Nutzung des erfindungsgemäßen Belagselementes als Fassadenelement, das dem Tageslicht ausgesetzt ist, ist auch eine Funktion als Energiequelle für weitere Verbraucher außerhalb des Belagselementes möglich. Als Energiespeicher ist neben einer Dünnschichtbatterie auch eine sogenannte micro energy cell (MEC) denkbar. Eine solche Mikrobatterie kann insbesondere in gedruckter Form vorgesehen sein. Die Ladung eines Energiespeichers kann beispielsweise durch elektrische Zuleitungen von außen oder durch ein piezoelektrisches und/oder ein photovoltaisches Element erfolgen. Dadurch wird benötigte elektrische Energie von dem Belagselement bzw. von der Funktionsschicht selbst bereitgestellt und steht aufgrund der Speicherung im Energiespeicher kontinuierlich zur Verfügung.

Insbesondere weist das Energieversorgungsmittel eine Induktionsspule auf. Vorzugsweise ist ebenfalls eine Induktionsfläche oder ein Induktionsbereich vorgesehen, so dass externe elektronische Geräte, wie Smartphones, Tablets, Computer oder dergleichen, kontaktlos durch Induktion durch das Belagselement geladen werden können. Hierfür ist es lediglich notwendig, die zu ladenden Geräte auf die Induktionsfläche, die dann als Ladefläche bzw. -bereich dient, abzulegen. Dies kann nach dem Qi- oder dem Powermat-Standard erfolgen.

Des Weiteren kann bei einer bevorzugten Ausgestaltung das Belagselement eine Bedienungseinrichtung aufweisen, wobei ein Bedienelement zur Eingabe und/oder Ausgabe von Daten vorgesehen sein kann. Das Bedienelement kann die Funktion einer Benutzerschnittstelle erfüllen. Über das Bedienelement kann beispielsweise eine neue Programmierung der Verarbeitungseinrichtung vorgenommen werden, indem Steuerdaten und/oder Sollwertvorgaben eingegeben werden. Durch eine Datenausgabe, insbesondere durch Anzeige mittels der Anzeigeeinrichtung, kann eine Rückmeldung an den Benutzer erfolgen, etwa zur Bestätigung eingegebener Daten oder wenn eine Datenübertragung erfolgreich abgeschlossen wurde. Vorzugsweise weist die Bedienungseinrichtung wenigstens einen Druckschalter und/oder ein kapazitives Element auf. Ein Druckschalter kann dabei einfache Schaltaufgaben übernehmen und insbesondere als Taster dienen. Durch ein kapazitives Element ergeben sich darüber hinaus weitere Möglichkeiten. Bei einem solchen Element lassen sich verschiedene Bereiche einer Eingabefläche zur Ausführung verschiedener Funktionen definieren. Zudem unterstützt ein kapazitives Element auch Bedienungsformen, die über die Funktionen eines reinen Drucktasters hinausgehen. So erlauben beispielsweise Wischgesten die Einstellung von Werten in Anlehnung an die Funktionsweise eines Schiebereglers anstelle der Eingabe eines konkreten Wertes. Dadurch kann etwa eine Dimmersteuerung für eine Beleuchtungseinrichtung oder ähnliches realisiert werden.

Das Energieversorgungsmittel kann ein Anschlussmittel zur elektrischen Kontaktierung eines elektrischen Verbrauchers, insbesondere der Funktionsschicht, und/oder einer insbesondere eine Induktionsspule aufweisenden, vorzugsweise als WLAN-, Bluetooth-, Qi- und/oder Powermat-Schnittstelle ausgebildeten Schnittstelle zur bevorzugten kabellosen Übertragung von Energie und/oder Informationen, insbesondere Daten, aufweisen. Das Anschlussmittel kann ferner als Kontaktfläche ausgebildet sein, insbesondere wobei eine kabellose Übertragung von Energie vorgesehen ist.

Darüber hinaus ist oberseitig auf dem Grundkörper und/oder der Funktionsschicht ein wenigstens einlagiger oberer Schichtaufbau vorgesehen. Der obere Schichtaufbau kann eine Dekorfolie und/oder ein Dekorpapier aufweisen und/oder zum Schutz der Funktionsschicht vorgesehen sein. Des Weiteren kann der obere Schichtaufbau auch durchsichtig ausgebildet sein. Erfindungsgemäß ist die Funktionsschicht unterhalb des Grundkörpers, dem oberen Schichtaufbau abgewandt, angeordnet.

Der grundsätzliche Aufbau des Belagselementes - abgesehen von der Funktionsschicht - kann analog zu bereits im Stand der Technik bekannten Belagselementen, insbesondere Belagselementen für Vinyl-Böden, modularen Böden oder Parkett, aufgebaut sein.

Des Weiteren kann die Funktionsschicht, insbesondere die Leiterplatte, auf den Grundkörper und/oder auf die Dekorfolie und/oder auf das Dekorpapier und/oder auf eine Trägerschicht aufgedruckt sein. Insbesondere kann die Funktionsschicht mittels Digitaldrucktechnik aufgedruckt sein. Dies vereinfacht den Schichtaufbau des erfindungsgemäßen Belagselementes und trägt zu einer Verringerung der Bauhöhe des Belagselementes bei. Weiterhin kann die Funktionsschicht auch separat von dem Grundkörper, der Dekorfolie, dem Dekorpapier und/oder der Trägerschicht dem Belagselement zur Verfügung gestellt werden. Beispielsweise kann die Funktionsschicht im Rahmen der Chip-on-Board-Technologie auf den Grundkörper, der Dekorfolie, dem Dekorpapier und/oder der Trägerschicht aufgebracht bzw. an diese angeordnet werden.

Wenigstens eine Gegenzugschicht unterhalb der Trägerschicht bzw. des Grundkörpers kann einer mechanischen Deformation des Belagselementes entgegenwirken. Durch zwei derartige Gegenzugschichten lässt sich beispielsweise bei einem Laminatboden eine Verformung vermeiden oder zumindest verringern. Bei zwei Gegenzugschichten müssen diese dabei nicht gleichartig ausgebildet sein, sondern können insbesondere unterschiedliche Schichtdicken und/oder Materialien aufweisen.

Insbesondere im Hinblick auf eine Verwendung des Belagselementes als Boden-, Wand- oder Deckenbelag wird das Verlegen bzw. allgemein die Montage des erfindungsgemäßen Belagselementes durch korrespondierende Nut-Feder-Verbindungsgeometrien an gegenüberliegenden Randseiten des Belagselementes vereinfacht. In diesem Zusammenhang hat das Prinzip einer sogenannten Klick-Verbindung weite Verbreitung gefunden. Mit Hilfe einer solchen Klick-Verbindungsgeometrie lassen sich die erfindungsgemäßen Belagselemente mit einem geringen personellen und technischen Aufwand verlegen.

Bei der erfindungsgemäßen Ausgestaltung des Belagselementes bzw. der korrespondierenden Verbindungsgeometrien von gegenüberliegenden Randseiten beinhaltet Verbindungsgeometrien, in deren Bereich keine Funktionsschicht angeordnet ist und/oder die letztlich frei von der Funktionsschicht sind.

Vorzugsweise ist das Belagselement, insbesondere oberseitig und/oder unterseitig, wasserfest, wasserdicht und/oder wasserabweisend ausgebildet. Die Wasserfestigkeit kann anhand des Einsatzzweckes ausgewählt werden. Insbesondere hält das Belagselement oberseitig und/oder unterseitig einer Wassersäule von größer 1000 mm, bevorzugt zwischen 1000 bis 50000 mm, weiter bevorzugt zwischen 5000 bis 10000 mm, stand. Die zuvor genannte Wassersäule ist nach der DIN EN 2081 bestimmt und gibt die hydrostatische Druckhöhe an, der ein Flächengebilde Stand hält. Sie ist dabei ein Maß für die Höhe des Widerstands, dem das Flächengebilde dem Durchdringen von Wasser entgegensetzt.

Des Weiteren betrifft die Erfindung ein System mit wenigstens einem Belagselement, insbesondere einem Belagselement nach einer der zuvor beschriebenen Ausführungsformen, einer Verarbeitungseinrichtung und wenigstens einem weiteren Belagselement, wobei das Belagselement einen Grundkörper und wenigstens eine Funktionsschicht aufweist, wobei die Funktionsschicht eine Übertragungseinrichtung zur Übertragung von Informationen und eine Messeinrichtung zur Erfassung von Informationen aufweist, wobei die Messeinrichtung derart mit der Übertragungseinrichtung gekoppelt ist, dass die Übertragungseinrichtung zur kabellosen Übertragung der von der Messeinrichtung empfangenen Informationen ausgebildet ist, wobei das Belagselement an allen Seitenrändern elektrisch kontaktierungslos und/oder elektrisch kontaktfrei ausgebildet ist und wobei die Verarbeitungseinrichtung und das Belagselement, insbesondere die Übertragungseinrichtung des Belagselementes, zum Informationsaustauch miteinander gekoppelt sind. Hinzuweisen ist darauf, dass das weitere Belagselement keine Funktionsschicht aufweist.

Erfindungsgemäß ist vorgesehen, dass jedes Belagselement derart ausgebildet ist, dass es mit der Verarbeitungseinrichtung kommunizieren und/oder Informationen austauschen kann. Ein Informationsaustausch der Belagselemente untereinander bzw. von dem Belagselement zu einem, insbesondere unmittelbar an ihm angeordneten, weiteren Belagselement ist erfindungsgemäß nicht vorgesehen. Hierfür kann das Belagselement an den Rändern elektrisch isolierend, zumindest aber auf jeden Fall elektrisch kontaktierungslos und/oder elektrisch kontaktfrei ausgebildet sein. Zur Gewährleistung des Informationsaustausches ist auch die Verarbeitungseinrichtung derart ausgebildet, dass sie Informationen an das Belagselement, insbesondere an die Übertragungseinrichtung und/oder die Messeinrichtung, übertragen kann. So kann eine Steuerung des Belagselementes und/oder eine Regelung mit Hilfe der Verarbeitungseinrichtung erfolgen. Dies ist gerade im Zusammenhang mit Smart-Home-Anwendungen durch einen Informationsaustausch zwischen der Verarbeitungseinrichtung und dem Belagselement vorteilhaft, da anhand der durch das Belagselement erfassten und an die Verarbeitungseinrichtung übertragenen Informationen eine mögliche Reaktion durch die Verarbeitungseinrichtung an weitere Geräte und insbesondere auch an das Belagselement zurückgegeben werden kann. So kann beispielsweise die Verarbeitungseinrichtung die Anzeigeeinrichtung des Belagselementes ansteuern und somit einer sich im Raum befindlichen Person Informationen vermitteln bzw. anzeigen.

Das erfindungsgemäße System bedingt ein Zusammenspiel zwischen dem Belagselement und einer, insbesondere externen, Verarbeitungseinrichtung, wobei die Verarbeitungseinrichtung als sogenannter Controller angesehen werden kann. Die Verarbeitungseinrichtung kann demgemäß die durch das wenigstens eine Belagselement gesendeten Signale empfangen sowie verarbeiten. Eine Weiterleitung und eine Kompatibilität eines Bussystems und/oder einer Smart-Home-Anwendung ist durch das erfindungsgemäße System möglich.

Verwendung kann das erfindungsgemäße System neben einer Anwendung im Bereich eines Smart-Homes auch in der Gesundheits- und der Sicherheitsbranche finden. Beispielsweise kann das erfindungsgemäße System erkennen, ob eine hilfsbedürftige Person auf dem Belagselement liegt und sich bewegt oder nicht, so dass von einem Sturz ausgegangen werden kann. Ebenfalls können Raumparameter bzw. eine physikalische Größe durch das erfindungsgemäße Belagselement überwacht werden und die Verarbeitungseinrichtung kann entsprechend der durch die Messeinrichtung des Belagselementes erfassten Informationen reagieren. So kann beispielsweise die Raumfeuchte, die Temperatur und/oder die Beleuchtungsstärke geregelt und/oder gesteuert werden. Zudem kann im Bereich eines Smart-Homes entsprechend der durch das Belagselement erfassten Informationen - durch Vorgabe der Verarbeitungseinrichtung - die Heizung und/oder die Jalousien geregelt und/oder gesteuert werden.

Durch das erfindungsgemäße System kann der Elektrosmog bei Bereitstellung einer elektrischen Funktionalität in einem Wand-, Boden- oder Deckenbelag deutlich reduziert werden, da nur einzelne Belagselemente anstelle des gesamten Belages bzw. der gesamten Belagselemente des Belages die erfindungsgemäße Funktionsschicht aufweisen. Dies ergibt zusätzlich einen Kostenvorteil, da das "autarke" Belagselement individuell sowie partiell verlegt werden kann, beispielsweise nur im Eingangsbereich. Weiterhin kann ein Raum über den jeweiligen Belag überwacht werden. Es ist keine aufwendige Kontaktierung zwischen einzelnen Belagselementen bzw. zwischen Belagselementen und weiteren Belagselementen notwendig, insbesondere da die Funktionsschicht nicht im Bereich der Nut-Feder-Verbindungsgeometrie vorgesehen ist.

Das erfindungsgemäße System kann sehr einfach bei einem bereits verlegten Belag integriert werden, indem das erfindungsgemäße Belagselement in den Belag eingesetzt und ein weiteres Belagselement gegen das erfindungsgemäße Belagselement ausgetauscht wird.

Bei einer besonders bevorzugten Ausführungsform der vorliegenden Erfindung ist eine Mehrzahl von Belagselementen vorgesehen, wobei die Belagselemente untereinander und/oder mit weiteren Belagselementen verbunden und/oder aneinander angeordnet sind. Erfindungsgemäß ist dabei vorgesehen, dass die Belagselemente elektrisch kontaktierungslos und/oder kontaktfrei miteinander verbunden sind, so dass kein Informationsaustausch über die Seitenränder des Belagselementes erfolgt. Jedes Belagselement kann für sich mit der Verarbeitungseinrichtung kommunizieren. Ein Informationsaustausch der Belagselemente untereinander kann erfindungsgemäß über die Verarbeitungseinrichtung erfolgen, die jegliche Informationen an die jeweiligen Belagselemente übertragen kann. Wie zuvor erläutert, ist die Anordnung an bzw. Verbindung mit weiteren Belagselementen, die keine erfindungsgemäße Funktionsschicht aufweisen, vorteilhaft, da so ein Belag bereitgestellt werden kann, der lediglich in einzelnen, partiellen Bereichen eine elektrische Funktionalität aufweist.

Vorzugsweise ist die Verarbeitungseinrichtung zur Decodierung der durch die Übertragungseinrichtung übertragenen und codierten Informationen ausgebildet. So können die Informationen in codierter Weise übertragen werden und eine Decodierung erfolgt erst am Bestimmungsort in der Verarbeitungseinrichtung. Eine codierte Informationsübertragung ist im Hinblick auf den Datenschutz sinnvoll, so dass eine Manipulation und/oder ein Datenmissbrauch vermieden werden kann. Eine Decodierung der Informationen ist innerhalb der Verarbeitungseinrichtung zur Analyse bzw. Verarbeitung der Informationen sinnvoll.

Weiterhin ist die Verarbeitungseinrichtung bevorzugt zur Positionserkennung des Belagselementes ausgebildet. Durch die Positionserkennung kann eine Zuordnung des Belagselementes zu seinem Standort erfolgen. So können beispielsweise ziel- und zweckgerichtet die Informationen im Verhältnis zu dem Standort des Belagselementes gesetzt werden. Hierfür kann das Belagselement auch seine Standortinformationen an die Verarbeitungseinrichtung übertragen und/oder die Verarbeitungseinrichtung kann anhand der jeweiligen Informationen des Belagselementes eine Zuordnung des Belagselementes und damit einhergehend eine Positionserkennung vornehmen. Dabei kann es sinnvoll sein, dass zuvor die Verarbeitungseinrichtung im Hinblick auf die Standortkoordinaten der jeweiligen Belagselemente kalibriert worden ist, so dass anschließend die Verarbeitungseinrichtung nach Empfangen der jeweiligen Informationen durch das Belagselement eine Zuordnung vornehmen kann. Gerade bei entsprechender Reaktion der Verarbeitungseinrichtung auf Informationen des Belagselementes, beispielsweise zur Steuerung und/oder Regelung weiterer Geräte im Bereich einer Smart-Home-Anwendung oder dergleichen, ist die Kenntnis des Standortes sinnvoll.

Eine Informationsübertragung ausgehend von dem Belagselement an die Verarbeitungseinrichtung kann kontinuierlich oder diskontinuierlich erfolgen. Eine diskontinuierliche Übertragung kann vorgesehen sein, wenn die Messeinrichtung abweichend vom Grundzustand Informationen aufnimmt, beispielsweise bei Aktivierung eines Piezoelementes durch eine Druckbelastung oder bei einer Änderung der Raumtemperatur. Folglich kann sowohl die Messeinrichtung als auch die Übertragungseinrichtung kontinuierlich oder diskontinuierlich die Messdaten übertragen. Demzufolge kann auch die Verarbeitungseinrichtung entweder kontinuierlich oder diskontinuierlich die jeweiligen Informationen verarbeiten und entsprechend auf diese reagieren. Eine diskontinuierliche Messwerterfassung sowie Messwertübertragung ist durch einen geringeren Energieverbrauch als eine kontinuierliche Messwertaufnahme und -übertragung gekennzeichnet. So können durch die diskontinuierliche Messwertaufnahme und -übertragung der Energieverbrauch des erfindungsgemäßen Belagselementes gesenkt werden.

Darüber hinaus kann bei einer weiteren bevorzugten Ausgestaltung die Verarbeitungseinrichtung zur Speicherung von Informationen ausgebildet sein. Dazu kann die Verarbeitungseinrichtung wenigstens ein Speichermittel aufweisen. Die gespeicherten Informationen können zum Vergleich mit empfangenen Informationen und/oder zur Verarbeitung vorgesehen sein. Folglich können Bewegungsabläufe erkannt werden. Ebenfalls ist ein Identifizieren und Navigieren von Personen sowie eine Personenverfolgung durch eine Speicherung der Informationen in der Verarbeitungseinrichtung möglich. Beispielsweise kann dies im Bereich des Marketings genutzt werden, um Benutzerverhalten sowie Besucherzahlen zu erkennen und zu analysieren. Demgemäß kann festgestellt werden, ob eine bestimmte Verkaufsfläche einen erhöhten Besucherandrang aufweist. Auch im Bereich des öffentlichen Dienstes kann eine Speicherung von Informationen sinnvoll sein, da so auf entsprechende Warteschlangen reagiert und die Anzahl der wartenden Personen erfasst werden kann.

Vorzugsweise sind Bewegungsmuster durch die Verarbeitungseinrichtung erstellbar, insbesondere vorgesehen zum Vergleich mit empfangenen Informationen. So kann ein Abgleich mit einem erwarteten Bewegungsablauf erfolgen und gegebenenfalls der Bewegungsablauf einer Person bzw. von Personen gesteuert und vorgegeben werden. Hierfür kann die Verarbeitungseinrichtung beispielsweise eine Anzeigeeinrichtung des Belagselementes ansteuern und somit den Personenstrom gezielt beeinflussen.

Zudem ist gemäß einer weiteren bevorzugten Ausgestaltung des Erfindungsgedankens vorgesehen, dass eine Steuereinrichtung, vorzugsweise ein Bussystem, und/oder ein elektronisches, insbesondere mobiles, Endgerät zum Empfangen von durch die Verarbeitungseinrichtung übertragenen Informationen vorgesehen ist. Die Steuereinrichtung kann der Verarbeitungseinrichtung zugeordnet sein. Demgemäß kann eine Steuerung von Geräten durch die Steuereinrichtung, die separat von der Verarbeitungseinrichtung vorgesehen sein kann, erfolgen.

Die Steuereinrichtung kann auch über die Verarbeitungseinrichtung Informationen, insbesondere vorgesehen zur Steuerung und/oder Regelung, an das Belagselement übertragen.

Das mobile Endgerät kann beispielsweise einer Privatperson zugeordnet sein, die zur Steuerung von Geräten, die mit der Steuereinrichtung gekoppelt sind, autorisiert ist. So kann beispielsweise die Raumtemperatur, die Beleuchtungsstärke und/oder der Feuchtigkeitsgehalt eines Raumes durch die Steuereinrichtung vorgegeben werden. Gleichfalls können durch die Steuereinrichtung auch die durch die Verarbeitungseinrichtung verarbeiteten Informationen einem Nutzer angezeigt werden; beispielsweise zu welchem Zeitpunkt welche Person den Raum betreten hat, die Bewegungsmuster von Besucherströmen und die Positionserkennung von Personen.

Im Bereich der Gesundheitsbranche ist daher möglich, dass dem Nutzer der Standort einer hilfsbedürftigen Person, die gestürzt sein kann, angezeigt werden kann. Eine Vermittlung der Informationen kann in visueller und/oder akustischer Form erfolgen.

Sowohl die Steuereinrichtung als auch das Endgerät können räumlich entfernt von der Verarbeitungseinrichtung vorgesehen sein und mobil durch den Nutzer zur kontinuierlichen Überwachung mitgeführt werden. So kann eine Einbruchsmeldung bei Registrierung eines unberechtigten Zugangs durch eine Person an die Steuereinrichtung und/oder das Endgerät übertragen werden.

Des Weiteren kann das Endgerät als Smartphone, Tablet, Laptop, Computer, Alarmeinrichtung und/oder Smart-Home-Applikation ausgebildet sein. Als Endgeräte sind ebenfalls eine Heizungsanlage, eine Lüftungsanlage, Jalousien und/oder Lampen, eine Klimaanlage oder dergleichen erfindungsgemäß möglich. Ebenfalls kann eine elektrisch steuerbare Tür als Endgerät vorgesehen sein.

Wie zuvor erläutert, kann die Steuereinrichtung und/oder das Endgerät zum Übertragen von Informationen an die Verarbeitungseinrichtung ausgebildet sein. Es ist ebenfalls oder alternativ möglich, dass das Endgerät Informationen des Belagselementes zur Steuerung und/oder Regelung empfängt.

Ferner können über die Steuereinrichtung und/oder das Endgerät die Verarbeitungseinrichtung, die Messeinrichtung, die Übertragungseinrichtung und/oder die Anzeigeeinrichtung steuerbar sein. Eine Steuerung der Anzeigeeinrichtung kann beispielsweise zur Informationsdarstellung vorgesehen sein. Weiterhin können durch Vorgabe der Steuereinrichtung auch die Endgeräte, die möglicherweise mit der Verarbeitungseinrichtung gekoppelt sind, gesteuert werden. Dabei kann vorgesehen sein, dass eine Vielzahl von Endgeräten vorhanden ist. Ferner kann ein Endgerät in Form eines Smartphones einem Nutzer zugeordnet sein und weitere Endgeräte, beispielsweise eine Heizungsanlage, eine Belüftungsanlage und/oder eine Alarmeinrichtung, können durch eine Steuereinrichtung geregelt und/oder gesteuert werden. Letztlich kann ebenfalls ein mobiles Endgerät weitere Endgeräte regeln und/oder steuern. Hierfür kann das mobile Endgerät beispielsweise Informationen an die Steuereinrichtung und/oder die Verarbeitungseinrichtung übertragen. Demgemäß können frühzeitig bei möglichem Alarm Sicherheitsmaßnahmen eingeleitet werden.

Alternativ oder zusätzlich kann auch vorgesehen sein, dass die Steuereinrichtung und/oder das Endgerät über die Verarbeitungseinrichtung, die Messeinrichtung, die Übertragungseinrichtung und/oder die Anzeigeeinrichtung steuerbar sind. Insbesondere sind die Steuereinrichtung und/oder das Endgerät über die Bedienungseinrichtung steuerbar. Demzufolge können auch die Verarbeitungseinrichtung, das Belagselement, die Messeinrichtung, die Übertragungseinrichtung und/oder die Anzeigeeinrichtung Informationen an die Steuereinrichtung und/oder das Endgerät übertragen.

Vorzugsweise sind die durch die Messeinrichtung erfassten Daten des Zugangskontrollmittels an die Verarbeitungseinrichtung, die Steuereinrichtung und/oder das Endgerät übertragbar. In den vorgenannten Einrichtungen können die durch das Zugangskontrollmittel erfassten Daten verarbeitet und insbesondere gespeichert werden. Auch ein Abgleich mit gespeicherten Daten, insbesondere zur Zuordnung bzw. Authentifizierung einer Person, ist durchführbar.

Bei einer besonders bevorzugten Ausgestaltung des Erfindungsgedankens ist die Übertragungseinrichtung, die Verarbeitungseinrichtung, die Steuereinrichtung und/oder das Endgerät mit einer Zugangskontrolleinrichtung gekoppelt. Eine Zugangskontrolleinrichtung kann die Schließanlage einer Sicherheitstür aufweisen oder einer Schließanlage zugeordnet sein, so dass die Sicherheitstür nach erfolgter Authentifizierung einer Person freigegeben werden kann. Grundsätzlich sind auch weitere Zugangskontrolleinrichtungen möglich, wie eine Video- und/oder Infrarotüberwachung, ein Drehkreuz, ein Tor und/oder ein Fahrstuhl. Eine Freigabe der Zugangskontrolleinrichtung kann nach Verarbeitung der durch das Zugangskontrollmittel übertragenen Daten erfolgen. So kann zunächst das Zugangskontrollmittel die Personenauthentifizierungsdaten an die Messeinrichtung übertragen, wobei nach Verarbeitung jener Daten - gegebenenfalls durch Abgleich mit gespeicherten Informationen - eine Freigabe erfolgen kann.

Insbesondere muss die Person, die über die Zugangskontrolleinrichtung Zugang zu einem abgetrennten Bereich erhalten möchte, das Zugangskontrollmittel nicht unmittelbar - wie bei in der Praxis bekannten Zugangskontrollen - vor ein Lesegerät halten. Erfindungsgemäß kann eine Zugangskontrolle mittels des Belagselementes und damit einhergehend mittels der Messeinrichtung in Kombination mit dem Zugangskontrollmittel, beispielsweise einer RFID-Karte, vorgesehen sein.

Ferner kann auch dem Belagselement ein Lesegerät zugeordnet sein, welches zum Auslesen des Zugangskontrollmittels ausgebildet ist. Das Belagselement und/oder der Belag kann ein Bewegungsprofil der durch das Lesegerät authentifizierten und/oder identifizierten Person erstellen, insbesondere wobei Informationen von dem Belagselement an das Lesegerät übertragen werden.

Grundsätzlich kann über die Bedienungseinrichtung des Belagselementes auch die Eingabe bzw. die Erfassung eines Identifizierungscodes erfolgen, der die Freigabe der Zugangskontrolleinrichtung zur Folge hat. Insbesondere kann eine Anzeigeeinrichtung zur Anzeige des Standortes der Funktionsschicht ausgebildet sein, so dass sich die Person in diesem Bereich des Belagselementes positionieren kann.

Darüber hinaus kann die Übertragungseinrichtung, die Verarbeitungseinrichtung, die Steuereinrichtung und/oder das Endgerät mit einer Alarmeinrichtung gekoppelt sein. Beispielsweise kann ein Alarm, vorzugsweise in visueller und/oder akustischer Form, bei Erkennung eines unautorisierten Zutritts zu dem gesicherten Bereich ausgelöst werden.

Insbesondere kann die Alarmeinrichtung auch Sicherheitspersonal, die Polizei und/oder die Feuerwehr alarmieren. Letztlich ist es auch möglich, die Alarmeinrichtung unabhängig von der Zugangskontrolleinrichtung in dem System vorzusehen. So kann beispielsweise die Alarmeinrichtung innerhalb eines gesicherten Bereiches, z. B. eines Museums, während des Tagesgeschäftes deaktiviert und nachts aktiviert werden, so dass beispielsweise nachts bei Betreten des gesicherten Bereiches ein Alarm ausgelöst werden kann. Die individuellen Vorgaben der Alarmeinrichtung können ziel- und zweckgerichtet an den jeweiligen Verwendungszweck angepasst werden.

Vorzugsweise ist die Alarmeinrichtung mit der Messeinrichtung gekoppelt. Auch eine Kopplung der Alarmeinrichtung mit dem Belagselement kann vorgesehen sein. Weiterhin kann die Alarmeinrichtung auch einen gesicherten Bereich absperren; vorzugsweise indem die Alarmeinrichtung mit einer Steuereinrichtung bzw. weiteren Endgeräten, wie einer Sicherheitstür, gekoppelt ist. So kann bei einem Alarm der gesicherte Bereich abgesperrt werden - durch zusätzlich zu ergreifende Maßnahmen.

Im Übrigen versteht es sich, dass in den vorgenannten Intervallen und Bereichsgrenzen jegliche Zwischenintervalle und Einzelwerte enthalten und als erfindungswesentlich offenbart anzusehen sind, auch wenn diese Zwischenintervalle und Einzelwerte nicht konkret angegeben sind.

Weitere Merkmale, Vorteile und Anwendungsmöglichkeiten der vorliegenden Erfindung ergeben sich aus der nachfolgenden Beschreibung von Ausführungsbeispielen anhand der Zeichnung und der Zeichnung selbst. Dabei bilden alle beschriebenen und/oder bildlich dargestellten Merkmale für sich oder in beliebiger Kombination den Gegenstand der vorliegenden Erfindung, unabhängig von ihrer Zusammenfassung in den Ansprüchen oder deren Rückbeziehung.

Es zeigt:
- Fig. 1: eine schematische Querschnittsansicht eines erfindungsgemäßen Belagselementes,
- Fig. 2: eine perspektivische schematische Darstellung eines Belags mit einem erfindungsgemäßen Belagselement,
- Fig. 3: eine perspektivische schematische Darstellung einer weiteren Ausführungsform eines erfindungsgemäßen Belagselementes,
- Fig. 4: eine perspektivische schematische Darstellung einer weiteren Ausführungsform eines erfindungsgemäßen Belagselementes,
- Fig. 5: eine schematische Darstellung des erfindungsgemäßen Systems,
- Fig. 6: eine schematische Darstellung einer weiteren Ausführungsform des erfindungsgemäßen Systems,
- Fig. 7: eine perspektivische schematische Darstellung einer weiteren Ausführungsform des erfindungsgemäßen Systems und
- Fig. 8: eine perspektivische schematische Darstellung einer weiteren Ausführungsform des erfindungsgemäßen Systems.

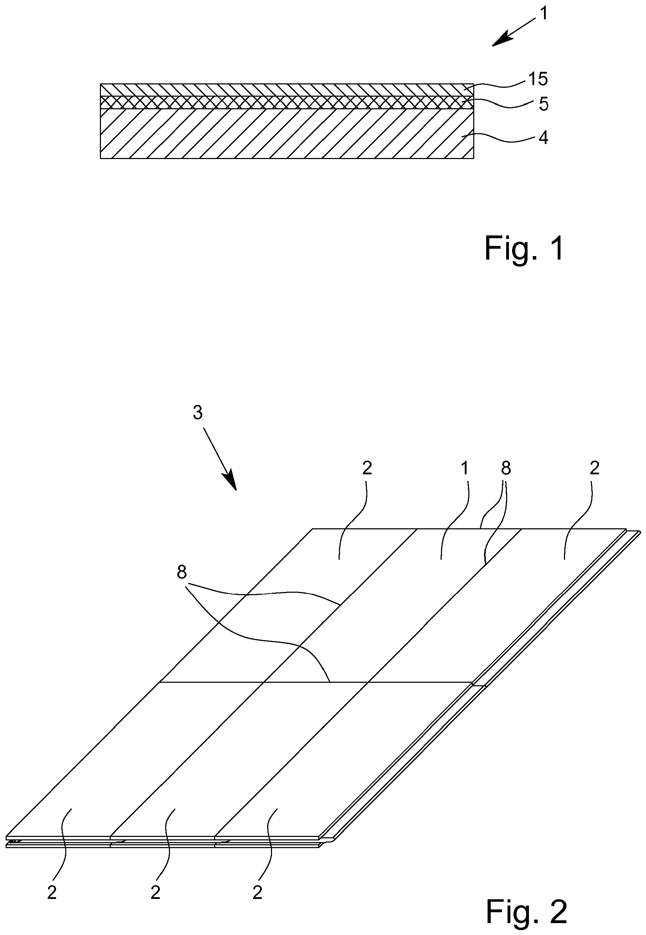

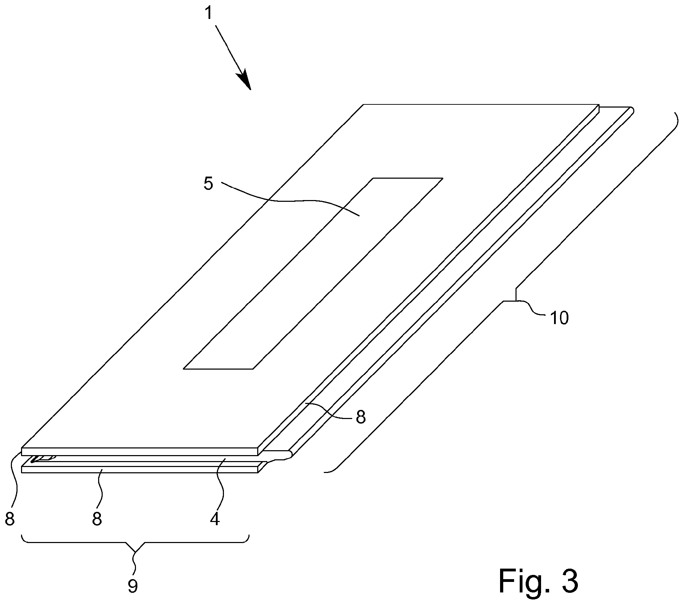

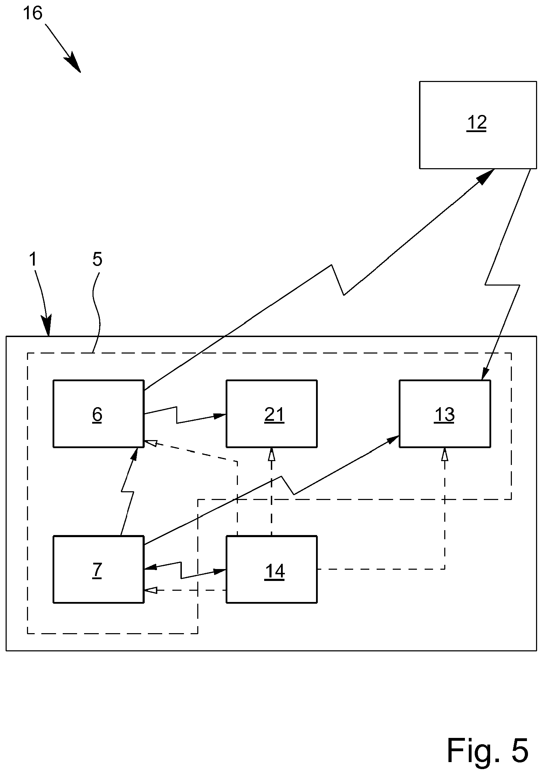

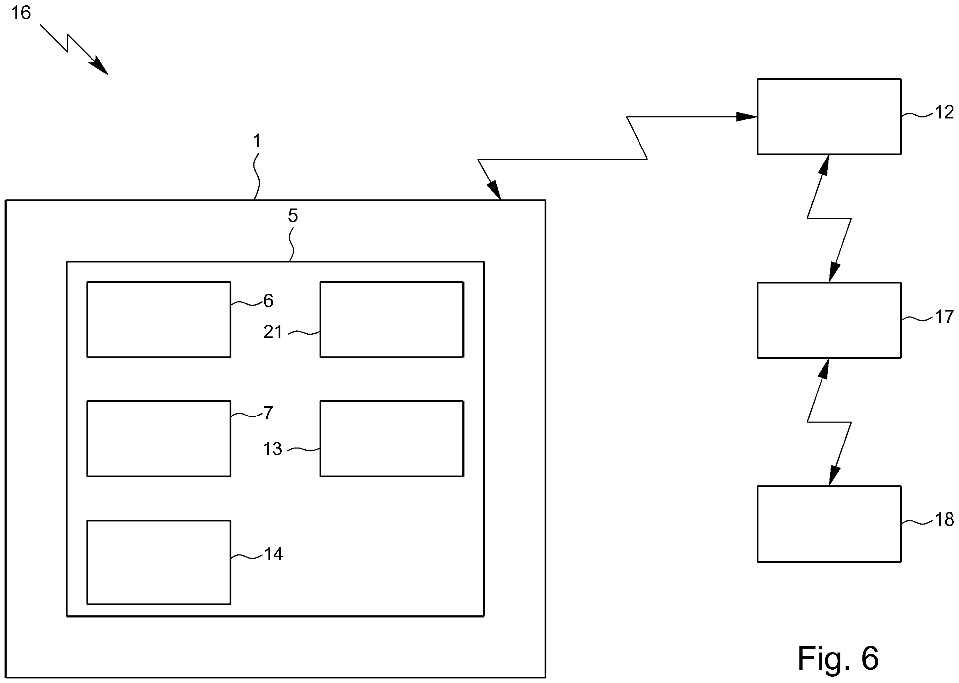

Fig. 1 zeigt ein Belagselement 1 mit einem Grundkörper 4 und einer Funktionsschicht 5, welches eine Übertragungseinrichtung 6 zur Übertragung von Informationen und eine Messeinrichtung 7 zur Erfassung von Informationen aufweist, wie dies aus Fig. 5 und 6 ersichtlich ist. Die Messeinrichtung 7 ist derart mit der Übertragungseinrichtung 6 gekoppelt, dass die Übertragungseinrichtung 6 zur, in weiteren Ausführungsformen kabellosen, Übertragung der von der Messeinrichtung 7 empfangenen Informationen ausgebildet ist. Fig. 3 zeigt, dass das Belagselement 1 an allen Seitenrändern 8 umlaufend elektrisch kontaktierungslos und elektrisch kontaktfrei ausgebildet ist.

Das Belagselement 1 kann innerhalb eines Belages 3, wie dies Fig. 2 zeigt, eingesetzt und mit weiteren Belagselementen 2 verbunden werden. Die weiteren Belagselemente 2 weisen keine Funktionsschicht 5 und demgemäß auch keine elektrische Funktionalität auf.

Weiterhin zeigt die Fig. 3, dass unter einer Funktionsschicht 5 der elektrisch leitende Bereich des Belagselementes 1 zu verstehen ist. Die Funktionsschicht 5 kann auf einer Trägerschicht angeordnet bzw. fest mit dieser verbunden sein, wobei die Trägerschicht oberseitig auf dem Grundkörper 4 vorgesehen sein kann. In der in Fig. 3 dargestellten Ausführungsform weist die Funktionsschicht 5 eine Leiterplatte auf.

Des Weiteren zeigt Fig. 3, dass die Funktionsschicht 5 zu wenigstens zwei Seitenrändern 8 beabstandet ist. In dem dargestellten Ausführungsbeispiel ist der Abstand mindestens 0,1 % der Breite 9 und der Länge 10 des Belagselementes 1. In dem dargestellten Ausführungsbeispiel ist der Abstand zu den Seitenrändern 8 in der Länge 10 etwa 20 % der Länge 10 des Grundkörpers 4 und der Abstand zu den Längsseiten beträgt etwa jeweils 35 % der Breite 9 des Belagselementes 1, wobei in dem dargestellten Ausführungsbeispiel die Funktionsschicht 5 zu allen Seitenrändern 8 beabstandet ist. In weiteren Ausführungsformen liegt der Abstand zwischen 0,1 % bis 40 % der Breite 9 und/oder der Länge 10 des Belagselementes 1.

Weiterhin ist in Fig. 3 gezeigt, dass die Funktionsschicht 5 mittig auf dem Grundkörper 4 vorgesehen ist. Zudem erstreckt sich die Funktionsschicht 5 in dem dargestellten Ausführungsbeispiel über etwa 35 % der Breite 9 des Belagselementes 1 und etwa über 45 % der Länge 10 des Belagselementes 1. In weiteren Ausführungsformen kann vorgesehen sein, dass sich die Funktionsschicht 5 zwischen 1 % bis 85 % der Breite 9 und/oder der Länge 10 des Belagselementes 1 auf den Grundkörper 4 erstreckt.

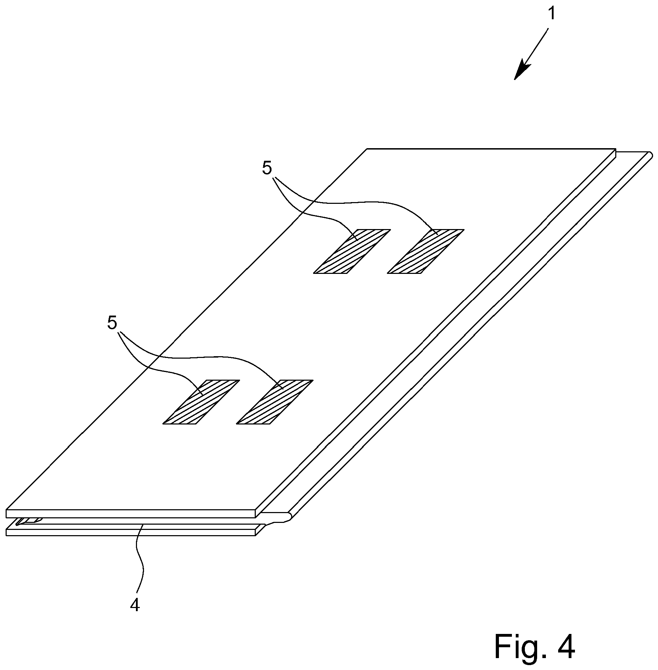

Fig. 4 zeigt, dass eine Mehrzahl von Funktionsschichten 5, in dem dargestellten Ausführungsbeispiel vier Funktionsschichten 5, vorgesehen sind. In dem dargestellten Ausführungsbeispiel sind die Funktionsschichten 5 unabhängig voneinander ausgebildet - das heißt, sie sind nicht miteinander gekoppelt. Sofern ein Zuschnitt des Belagselementes 1 zur Anpassung an einen Untergrund erfolgen soll, können einzelne Funktionsschichten 5 gegebenenfalls abgetrennt werden, wobei zur Gewährleistung der elektrischen Funktionalität weiterhin wenigstens eine Funktionsschicht 5 an dem Belagselement 1 bzw. an dem Grundkörper 4 angeordnet sein sollte.

Des Weiteren ist aus Fig. 4 ersichtlich, dass die Funktionsschichten 5 voneinander beabstandet angeordnet sind. Der Abstand bzw. Freiraum kann beispielsweise bei Zuschnitt des Belagselementes 1 als Schnittbereich dienen.

In Fig. 5 ist eine schematische Darstellung des Systems 16 und ebenfalls des Belagselementes 1 gezeigt. Aus der Darstellung geht hervor, dass die Übertragungseinrichtung 6 zur Verarbeitung der von der Messeinrichtung 7 übertragenen Informationen ausgebildet ist. Nicht dargestellt ist, dass die Übertragungseinrichtung 6 wenigstens eine Antenne zum Senden und/oder Empfangen von Informationen aufweisen kann. Die Informationen können über elektromagnetische Wellen übertragbar sein. Zudem ist nicht dargestellt, dass die Übertragungseinrichtung 6 zur Codierung und/oder Verschlüsselung der Informationen ausgebildet sein kann, was gegebenenfalls durch Verarbeitung der durch die Messeinrichtung 7 empfangenen Informationen erfolgen kann.

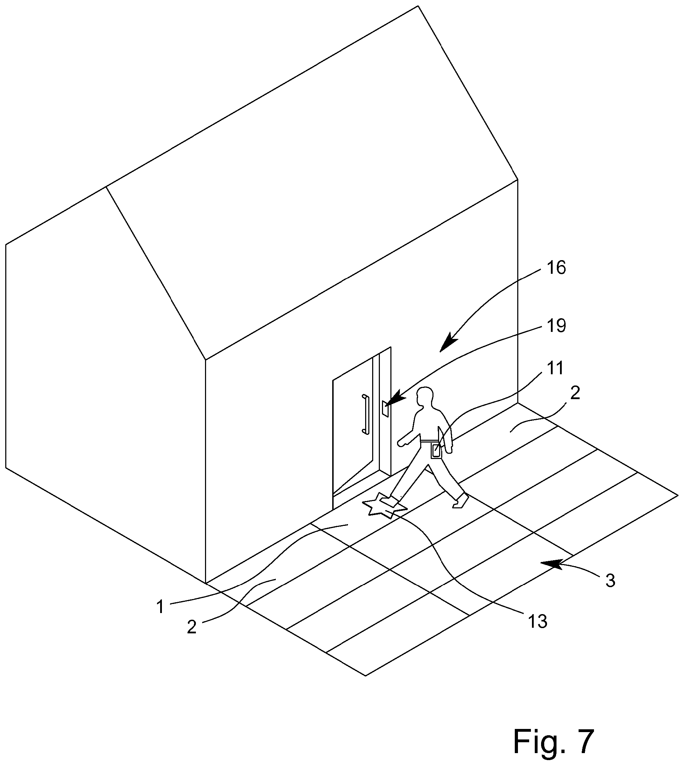

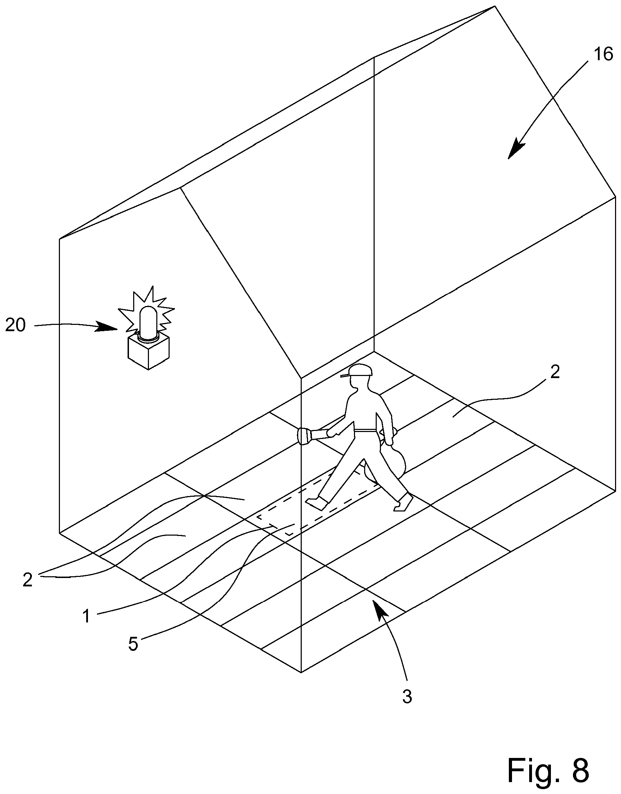

Die Fig. 7 und 8 zeigen, dass die Messeinrichtung 7 wenigstens einen Sensor aufweisen kann. In dem in Fig. 8 dargestellten Ausführungsbeispiel weist die Messeinrichtung 7 einen Sensor zur Messung von Bewegungen auf. Als Bewegungssensor dient in dem dargestellten Ausführungsbeispiel ein Drucksensor, der auf einem piezoelektrischen Effekt beruht. In weiteren, nicht dargestellten Ausführungsformen kann ein Sensor zur Messung von Temperatur, Schwingungen und/oder als Näherungssensor, Wärmesensor und/oder Schwingungssensor ausgebildet sein.

Zudem ist in Fig. 7 gezeigt, dass die Messeinrichtung 7 der Funktionsschicht 5 des Belagselementes 1 zum Auslesen eines Zugangskontrollmittels 11, in dem dargestellten Ausführungsbeispiel eine RFID-Karte, ausgebildet ist. In weiteren, nicht dargestellten Ausführungsformen kann die Messeinrichtung 7 zum Empfangen eines Taktsignals, von Energie und/oder Personenauthentifizierungsdaten eines Zugangskontrollmittels 11 ausgebildet sein. In dem dargestellten Ausführungsbeispiel erfolgt ein Zugang zu einem gesicherten Bereich durch ein Auslesen des Zugangskontrollmittels 11 durch das Belagselement 1, welches die sich zu authentifizierende Person betritt.

Nicht dargestellt ist, dass ein Lesegerät zum Auslesen des Zugangskontrollmittels 11 dem Belagselement 1 zugeordnet sein kann. Insbesondere kann eine Authentifizierng bzw. Identifizierung der Person durch das Lesegerät erfolgen, wobei ein Bewegungsprofil der Person nach erfolgter Authentifizierung bzw. Identifizierung durch das Belagselement 1, vorzugsweise durch die Messeinrichtung 7, erstellbar ist.

Nicht dargestellt ist, dass die Messeinrichtung 7 zur Digitalwandlung gemessener, analoger Informationen in digitale Informationen ausgebildet ist. So können digitale Informationen an die Übertragungseinrichtung 6 übertragen werden. Darüber hinaus kann auch die Übertragungseinrichtung 6 zur Digitalwandlung von analogen Informationen der Messeinrichtung 7 ausgebildet sein.

Darüber hinaus zeigen die Fig. 5 und 6, dass die Funktionsschicht 5 eine Speichereinrichtung 21 zur Speicherung der durch die Übertragungseinrichtung 6 verarbeiteten bzw. empfangenen Informationen aufweist. Die Speichereinrichtung 21 kann mit der Messeinrichtung 7 und der Übertragungseinrichtung 6 gekoppelt sein. Fig. 5 zeigt, dass der Speichereinrichtung 21 Informationen von der Übertragungseinrichtung 6 übertragen werden.

Im Übrigen zeigen die Fig. 5 und 6, dass der Funktionsschicht 5 eine Verarbeitungseinrichtung 12 zum Empfangen von durch die Übertragungseinrichtung 6 übertragenen Informationen zugeordnet ist. Die Verarbeitungseinrichtung 12 kann zudem separat von dem Belagselement 1 vorgesehen sein. In Fig. 6 ist gezeigt, dass die Informationen kabellos übertragen werden können.

Fig. 5 zeigt außerdem, dass das Belagselement 1 sowie die Funktionsschicht 5 eine Anzeigeeinrichtung 13 aufweisen. Die Anzeigeeinrichtung 13 kann zur akustischen, taktilen, haptischen, olfaktometrischen und/oder visuellen Vermittlung von Informationen ausgebildet sein. In dem in Fig. 7 dargestellten Ausführungsbeispiel ist die Anzeigeeinrichtung 13 oberseitig auf dem Grundkörper 4 angeordnet und als Leuchtschicht ausgebildet. Die Anzeigeeinrichtung 13 vermittelt im dargestellten Ausführungsbeispiel Informationen in visueller Form, in vorliegendem Fall zur korrekten Positionierung zur Personenauthentifizierung.

Ferner zeigen die Fig. 5 und 6, dass der Funktionsschicht 5 wenigstens ein Energieversorgungsmittel 14 zugeordnet ist. In Fig. 6 weist die Funktionsschicht 5 das Energieversorgungsmittel 14 auf. In Fig. 5 ist das Energieversorgungsmittel 14 zwar in dem Belagselement 1 vorgesehen, jedoch nicht innerhalb der Funktionsschicht 5. In einer nicht dargestellten Ausführungsform weist das Energieversorgungsmittel 14 einen Energiespeicher, beispielsweise eine Dünnschichtbatterie, auf. Darüber hinaus kann das Energieversorgungsmittel 14 zum Empfangen von Energie durch die Messeinrichtung 7 ausgebildet sein. Zudem kann der Messeinrichtung 7 Energie übertragen oder in ihr Energie erzeugt werden und diese Energie, insbesondere elektrische Energie, kann an das Energieversorgungsmittel 14 und an den Energiespeicher zur Energieversorgung des Belagselementes 1 übertragen werden. Die Messeinrichtung 7 kann in einem nicht dargestellten Ausführungsbeispiel ein Piezoelement aufweisen und damit einhergehend bei einer Druckbelastung eine Spannung erzeugen. Weiterhin kann auch das Energieversorgungsmittel 14 ein photovoltaisches Element aufweisen, insbesondere bei Sonneneinstrahlung auf das Belagselement 1.

Im Übrigen ist in den dargestellten Ausführungsbeispielen nicht dargestellt, dass das Energieversorgungsmittel 14 ein Anschlussmittel zur elektrischen Kontaktierung eines elektrischen Verbrauchers aufweisen kann. Als elektrischer Verbraucher kann beispielsweise die Funktionsschicht 5, aber auch weitere externe Verbraucher, beispielsweise ein Smartphone, welche auf das Belagselement 1 gelegt werden, vorgesehen sein. Des Weiteren kann das Energieversorgungsmittel 14 eine Induktionsspule und darüber hinaus eine als WLAN-, Bluetooth-, Qi- und/oder Powermat-Schnittstelle ausgebildete Schnittstelle zur Übertragung von Energie und/oder Informationen aufweisen. Diese Übertragung kann in weiteren Ausführungsformen kabellos erfolgen. Letztlich versteht es sich, dass das Energieversorgungsmittel 14 auch per Induktion ladbar ist. Grundsätzlich kann das Energieversorgungsmittel 14 per Induktion einen elektrischen Verbraucher laden als auch selbst durch Induktion geladen werden.

Im Übrigen zeigt Fig. 1, dass oberseitig auf dem Grundkörper 4 und oberseitig auf der Funktionsschicht 5 ein wenigstens einlagiger oberer Schichtaufbau 15 vorgesehen ist. Der obere Schichtaufbau 15 ist im dargestellten Ausführungsbeispiel die Benutzungsseite und dient somit auch zum Schutz der Funktionsschicht 5. Nicht dargestellt ist, dass der obere Schichtaufbau 15 eine Dekorfolie und/oder ein Dekorpapier aufweisen kann. In weiteren Ausführungsformen kann der obere Schichtaufbau 15 zumindest teilweise transparent und/oder transluzent ausgebildet sein, so dass die Anzeigeeinrichtung 13 sichtbar ist.

Nicht dargestellt ist, dass die Funktionsschicht 5 auch unterhalb des Grundkörpers 4, dem oberen Schichtaufbau 15 abgewandt angeordnet sein kann.

Des Weiteren ist nicht dargestellt, dass die Funktionsschicht 5 auf den Grundkörper 4, auf die Dekorfolie, auf das Dekorpapier und/oder auf eine Trägerschicht aufgedruckt sein kann. Als Druckverfahren würde sich beispielsweise das Digitaldruckverfahren eignen.

Das in Fig. 3 dargestellte Belagselement 1 ist wasserabweisend ausgebildet. In weiteren Ausführungsformen kann das Belagselement 1 wasserfest sowie wasserdicht ausgebildet sein. Die Wasserfestigkeit kann oberseitig und/oder unterseitig des Belagselements 1 vorgesehen sein. Das Belagselement 1 kann zudem in weiteren Ausführungsformen oberseitig und/oder unterseitig einer Wassersäule von größer 1000 mm und in weiteren Ausführungsformen zwischen 5000 bis 10000 mm standhalten. Die Wasserfestigkeit kann durch eine Versiegelung des Grundkörpers 4 bzw. der Ober- und der Unterseite des Grundkörpers 4 durch den wenigstens einlagigen oberen Schichtaufbau 15 erfolgen.

In Fig. 4 ist gezeigt, dass das Belagselement 1 an allen seinen Seitenrändern 8 sogenannte Nut-Feder-Verbindungsgeometrien aufweist. Die Funktionsschicht 5 ragt dabei in dem in Fig. 3 und 4 dargestellten Ausführungsbeispiel nicht in den Bereich der Klick-Verbindung bzw. der Nut-Feder-Verbindungsgeometrie. Dies ergibt sich durch die Beabstandung der Funktionsschicht 5 zu den Seitenrändern 8.

Ferner ist in Fig. 5 schematisch das System 16 mit wenigstens einem Belagselement 1 gezeigt. Das System 16 weist eine Verarbeitungseinrichtung 12 auf. In Fig. 7 ist gezeigt, dass das System 16 wenigstens ein weiteres Belagselement 2 aufweist, wobei das weitere Belagselement 2 keine elektrische Funktionalität beinhaltet. Das Belagselement 1 weist einen Grundkörper 4 und wenigstens eine Funktionsschicht 5 auf, wobei die Funktionsschicht 5 eine Übertragungseinrichtung 6 zur Übertragung von Informationen und eine Messeinrichtung 7 zur Erfassung von Informationen aufweist. Die Messeinrichtung 7 ist derart mit der Übertragungseinrichtung 6 gekoppelt, dass die Übertragungseinrichtung 6 zur Übertragung der von der Messeinrichtung 7 empfangenen Informationen ausgebildet ist. Das Belagselement 1 ist an allen Seitenrändern 8 elektrisch kontaktierungslos und/oder elektrisch kontaktfrei ausgebildet, wobei die Verarbeitungseinrichtung 12 und das Belagselement 1 zum Informationsaustausch miteinander gekoppelt sind. In dem in Fig. 5 dargestellten Ausführungsbeispiel ist die Verarbeitungseinrichtung 12 mit der Übertragungseinrichtung 6 des Belagselements 1 gekoppelt.

In den dargestellten Ausführungsbeispielen ist nicht gezeigt, dass eine Mehrzahl von Belagselementen 1 in dem System 16 in einem Belag 3 vorgesehen sein kann. Bei einer Mehrzahl von Belagselementen 1 ist vorgesehen, dass jedes Belagselement 1 mit der Verarbeitungseinrichtung 12 kommuniziert und Informationen austauschen kann. Das Belagselement 1 ist in dem dargestellten Ausführungsbeispiel mit weiteren Belagselementen 2 verbunden.

Darüber hinaus ist nicht dargestellt, dass die Verarbeitungseinrichtung 12 zur Decodierung der durch die Übertragungseinrichtung 6 übertragenen und codierten Informationen ausgebildet ist. Nach der Decodierung der Informationen kann in der Verarbeitungseinrichtung 12 eine Verarbeitung erfolgen, die beispielsweise zur Positionserkennung des Belagselementes 1 führt. Zur Positionserkennung des Belagselementes 1 kann dieses von seiner Einbaulage zuvor kalibriert worden sein, so dass beim Empfangen der Informationen die Verarbeitungseinrichtung 12 dem Belagselement 1 einen Standort zuordnen kann. Des Weiteren kann die Verarbeitungseinrichtung 12 zur Speicherung von Informationen ausgebildet sein, so dass beispielsweise gespeicherte Informationen zum Vergleich mit empfangenen Informationen und/oder zur Verarbeitung vorgesehen sind.

Zudem können Bewegungsmuster durch die Verarbeitungseinrichtung 12 erstellbar sein, welche beispielsweise zum Vergleich mit empfangenen Informationen vorgesehen sind.

Fig. 6 zeigt, dass eine Steuereinrichtung 17 sowie ein elektronisches Endgerät 18 zum Empfangen von Informationen, die durch die Verarbeitungseinrichtung 12 übertragbar sind, vorgesehen ist. In weiteren Ausführungsformen kann die Steuereinrichtung 17 ein Bussystem aufweisen. Das Endgerät 18 kann in weiteren Ausführungsformen ein mobiles Endgerät 18 sein. Auch Geräte, die mit einer Smart-Home-Anwendung gekoppelt sind bzw. im Rahmen eines Smart-Home-Konzeptes vorgesehen sind, wie beispielsweise eine Heizungseinrichtung, eine Lüftungseinrichtung und/oder eine Beleuchtungseinrichtung, sind als Endgeräte 18 möglich.

Als Endgerät 18 kann in weiteren Ausführungsformen auch ein Smartphone, Tablet, Laptop, Computer, eine Alarmeinrichtung und/oder eine Smart-Home-Applikation vorgesehen sein.

Weiterhin ist in Fig. 6 gezeigt, dass die Steuereinrichtung 17 und das Endgerät 18 zum Übertragen von Informationen an die Verarbeitungseinrichtung 12 ausgebildet sind. Ebenfalls können die Steuereinrichtung 17 und das Endgerät 18 durch die Verarbeitungseinrichtung 12 und darüber hinaus auch durch Informationen der Messeinrichtung 7, der Übertragungseinrichtung 6 und/oder Anzeigeeinrichtung 13, die an die Verarbeitungseinrichtung 12 übertragen werden, gesteuert und/oder geregelt werden. Darüber hinaus kann auch die Steuereinrichtung 17 und das Endgerät 18 die Verarbeitungseinrichtung 12, die Messeinrichtung 7, die Übertragungseinrichtung 6 sowie die Anzeigeeinrichtung 13 steuern bzw. regeln. In Fig. 5 ist gezeigt, dass Informationen von der Verarbeitungseinrichtung 12 direkt an die Anzeigeeinrichtung 13 übertragen werden können. In Fig. 6 ist eine Datenübertragung von der Verarbeitungseinrichtung 12 an das Belagselement 1 vorgesehen.

In dem in Fig. 7 und 8 dargestellten Ausführungsbeispiel wird das Belagselement 1 im Sicherheitsbereich eingesetzt. Hierfür sind die durch die Messeinrichtung 7 erfassten Daten des Zugangskontrollmittels 11 an die Verarbeitungseinrichtung 12, die Steuereinrichtung 17 und/oder das Endgerät 18 übertragbar. In den vorgenannten Einrichtungen 12, 17, 18 können die Personenauthentifizierungsdaten des Zugangskontrollmittels 11 verarbeitet und mit gespeicherten Daten abgeglichen werden.

Fig. 7 zeigt, dass die Übertragungseinrichtung 6, die Verarbeitungseinrichtung 12, die Steuereinrichtung 17 und das Endgerät 18 mit einer Zugangskontrolleinrichtung 19 gekoppelt sind. Eine Freigabe der Zugangskontrolleinrichtung 19 kann nach Verarbeitung der durch das Zugangskontrollmittel 11 übertragenen Dateninformationen vorgesehen sein. In dem dargestellten Ausführungsbeispiel ist die Zugangskontrolleinrichtung 19 im Bereich einer Sicherheitstür angeordnet und kontrolliert den Schließmechanismus bzw. die Schließanlage der Sicherheitstür. Bei erfolgter Freigabe kann ein Öffnen der Tür einleitbar sein.

Eine Alarmeinrichtung 20, die mit dem Belagselement 1 gekoppelt ist, ist in Fig. 8 gezeigt. Die Alarmeinrichtung 20 ist sowohl mit der Übertragungseinrichtung 7, der Verarbeitungseinrichtung 12 und der Steuereinrichtung 17 und in weiteren Ausführungsformen mit dem Endgerät 18 gekoppelt. Die Alarmeinrichtung 20 ist derart ausgebildet, dass bei nicht vorhandener Freigabe der Zugangskontrolleinrichtung 19 beim unbefugten Betreten eines gesicherten Bereichs ein Alarm ausgelöst wird. Der Alarm kann beispielsweise in visueller und akustischer Form, wie im dargestellten Ausführungsbeispiel, erfolgen und gleichzeitig auch eine Alarmierung von nicht dargestelltem Sicherheitspersonal auslösen.

Die Alarmeinrichtung 20 kann in weiteren Ausführungsformen aktiviert und deaktiviert werden, so dass bei Benutzung des Raumes durch mehrere Personen eine Deaktivierung der Alarmeinrichtung 20 erfolgen kann. Sofern der Raum gesichert werden und kein unautorisierter Zutritt erfolgen soll, so kann die Alarmeinrichtung 20 aktiviert werden und bei jeglicher Registrierung einer Bewegung bzw.

Temperaturänderung im Raum kann das Belagselement 1, insbesondere über die Verarbeitungseinrichtung 12, Informationen an die Alarmeinrichtung 20 übertragen. Hierzu kann in weiteren Ausführungsformen die Alarmeinrichtung 20 mit der Messeinrichtung 7 gekoppelt sein.

### Bezugszeichenliste:

- 1: Belagselement
- 2: weiteres Belagselement
- 3: Belag
- 4: Grundkörper
- 5: Funktionsschicht
- 6: Übertragungseinrichtung
- 7: Messeinrichtung
- 8: Seitenränder
- 9: Breite
- 10: Länge
- 11: Zugangskontrollmittel
- 12: Verarbeitungseinrichtung
- 13: Anzeigeeinrichtung
- 14: Energieversorgungsmittel
- 15: oberer Schichtaufbau
- 16: System
- 17: Steuereinrichtung
- 18: elektronisches Endgerät
- 19: Zugangskontrolleinrichtung
- 20: Alarmeinrichtung
- 21: Speichereinrichtung

## Patentansprüche

1. Belagselement (1) zur Verwendung als Fußboden-, Wand- und/oder Deckenbelag, mit einem Grundkörper (4) und wenigstens einer Funktionsschicht (5), wobei die Funktionsschicht (5) eine Übertragungseinrichtung (6) zur Übertragung von Informationen und eine Messeinrichtung (7) zur Erfassung von Informationen aufweist, wobei die Messeinrichtung (7) derart mit der Übertragungseinrichtung (6) gekoppelt ist, dass die Übertragungseinrichtung (6) zur kabellosen Übertragung der von der Messeinrichtung (7) empfangenen Informationen ausgebildet ist, wobei das Belagselement (1) an allen Seitenrändern (8) umlaufend elektrisch kontaktierungslos und elektrisch kontaktfrei ausgebildet ist, wobei die Funktionsschicht (5) zu wenigstens zwei Seitenrändern (8) beabstandet ist,
**dadurch gekennzeichnet,**
**dass** die Funktionsschicht (5) unterhalb des Grundkörpers (4), einem oberen Schichtaufbau (15) abgewandt, angeordnet ist und wobei korrespondierende Nut-Feder-Verbindungsgeometrien an gegenüberliegenden Randseiten des Belagselements (1) vorgesehen sind.

2. Belagselement nach Anspruch 1, **dadurch gekennzeichnet, dass** der Abstand der Funktionsschicht (5) zu den wenigstens zwei Seitenrändern (8) mindestens 0,1% der Breite und/oder Länge des Belagselementes ist, bevorzugt zwischen 0,1% bis 40%, weiter bevorzugt zwischen 1% bis 20% liegt, und/oder dass die Funktionsschicht (5) mittig auf dem Grundkörper (4) angeordnet ist und/oder dass sich die Funktionsschicht (5) zwischen 1% bis 85%, bevorzugt zwischen 5% bis 75%, weiter bevorzugt zwischen 10% bis 50% und insbesondere zumindest im Wesentlichen 20%, der Breite (9) und/oder Länge (10) des Belagselementes (1) auf dem Grundkörper (4) erstreckt.

3. Belagselement nach Anspruch 1 oder 2, **dadurch gekennzeichnet, dass** die Funktionsschicht (5) als eine elektrisch leitfähige Leiterplatte ausgebildet ist und/oder dass die Funktionsschicht (5) auf einer Trägerschicht angeordnet ist.

4. Belagselement nach einem der vorhergehenden Ansprüche, **dadurch gekennzeichnet, dass** die Funktionsschicht (5) als elektrisch leitende Funktionsschicht (5) ausgebildet ist.

5. Belagselement nach einem der vorhergehenden Ansprüche, **dadurch gekennzeichnet, dass** eine Mehrzahl von Funktionsschichten (5) vorgesehen ist.

6. Belagselement nach einem der vorhergehenden Ansprüche, **dadurch gekennzeichnet, dass** die Übertragungseinrichtung (6) wenigstens eine Antenne zum Senden und/oder Empfangen von Informationen aufweist.

7. Belagselement nach einer der vorhergehenden Ansprüche, **dadurch gekennzeichnet, dass** die Übertragungseinrichtung (6) zur Verarbeitung der von der Messeinrichtung (7) übertragenen Informationen ausgebildet ist, insbesondere wobei die Übertragungseinrichtung (6) zur Codierung und/oder Verschlüsselung der Informationen ausgebildet ist.

8. Belagselement nach einem der vorhergehenden Ansprüche, **dadurch gekennzeichnet, dass** die Messeinrichtung (7) wenigstens einen Sensor aufweist und/oder dass die Messeinrichtung (7) zum Auslesen eines Zugangskontrollmittels (11) ausgebildet ist.

9. Belagselement nach einem der vorhergehenden Ansprüche, **dadurch gekennzeichnet, dass** der Funktionsschicht (5) eine Verarbeitungseinrichtung (12) zum Empfangen von durch die Übertragungseinrichtung (6) übertragenen Informationen zugeordnet ist.

10. Belagselement nach einem der vorhergehenden Ansprüche, **dadurch gekennzeichnet, dass** der Funktionsschicht (5) wenigstens ein Energieversorgungsmittel (14) zugeordnet ist und/oder in die Funktionsschicht (5) integriert ist.

11. System (16) **gekennzeichnet durch** wenigstens ein erstes Belagselement (1) nach einem der
vorhergehenden Ansprüche, eine Verarbeitungseinrichtung (12) und wenigstens ein weiteres zweites
Belagselement (2), wobei das erste Belagselement (1) einen Grundkörper (4) und wenigstens eine Funktionsschicht (5) aufweist, wobei die Funktionsschicht (5) eine Übertragungseinrichtung (6) zur Übertragung von Informationen und eine Messeinrichtung (7) zur Erfassung von Informationen aufweist, wobei die Messeinrichtung (7) derart mit der Übertragungseinrichtung (6) gekoppelt ist, dass die Übertragungseinrichtung (6) zur kabellosen Übertragung der von der Messeinrichtung (7) empfangenen Informationen ausgebildet ist, wobei das erste Belagselement (1) an allen Seitenrändern (8) elektrisch kontaktierungslos und elektrisch kontaktfrei ausgebildet ist, wobei die Verarbeitungseinrichtung (12) und das erste Belagselement (1) zum Informationsaustausch miteinander gekoppelt sind und wobei eine Mehrzahl von ersten Belagselementen (1) vorgesehen ist, wobei die ersten Belagselemente (1) untereinander und/oder mit weiteren zweiten Belagselementen (2) randseitig mittels Nut-Feder-Verbindungen verbunden sind.

12. System nach Anspruch 11, **dadurch gekennzeichnet, dass** die Verarbeitungseinrichtung (12) zur Decodierung der durch die Übertragungseinrichtung (6) übertragenen und codierten Informationen ausgebildet ist und/oder dass die Verarbeitungseinrichtung (12) zur Positionserkennung des ersten Belagselementes (1) ausgebildet ist.

13. System nach einem der Ansprüche 11 oder 12, **dadurch gekennzeichnet, dass** eine Steuereinrichtung (17) und/oder ein elektronisches Endgerät (18) zum Empfangen von durch die Verarbeitungseinrichtung (12) übertragenen Informationen vorgesehen ist.

14. System nach einem der Ansprüche 11 bis 13, **dadurch gekennzeichnet, dass** die Verarbeitungseinrichtung (12), die Messeinrichtung (7), die Übertragungseinrichtung (6) und/oder die Anzeigeeinrichtung (13) über die Steuereinrichtung (17) und/oder das Endgerät (18) steuerbar sind.

15. System nach einem der Ansprüche 11 bis 14, **dadurch gekennzeichnet, dass** die durch die Messeinrichtung (7) erfassten Daten des Zugangskontrollmittels (11) an die Verarbeitungseinrichtung (12) und/oder die Steuereinrichtung (17) und/oder das Endgerät (18) übertragbar sind.

16. System nach einem der Ansprüche 11 bis 15, **dadurch gekennzeichnet, dass** die Übertragungseinrichtung (6), die Verarbeitungseinrichtung (12), die Steuereinrichtung (17) und/oder das Endgerät (18) mit einer Zugangskontrolleinrichtung (19) gekoppelt sind.

17. System nach einem der Ansprüche 11 bis 16, **dadurch gekennzeichnet, dass** die Übertragungseinrichtung (6), die Verarbeitungseinrichtung (12), die Steuereinrichtung (17) und/oder das Endgerät (18) mit einer Alarmeinrichtung (20) gekoppelt sind.

## Claims

1. Covering element (1) for use as a floor, wall and/or ceiling covering, having a main body (4) and at least one functional layer (5), wherein the functional layer (5) has a transmission device (6) for transmitting information and a measuring device (7) for detecting information, wherein the measuring device (7) is coupled to the transmission device (6) in such a manner in that the transmission device (6) is designed for the wireless transmission of the information received from the measuring device (7), wherein the covering element (1) is designed to be electrically contactless and electrically contact-free all the way around on all the side edges (8), wherein the functional layer (5) is spaced apart from at least two side edges (8),
**characterized in that**
the functional layer (5) is arranged below the main body (4), facing away from an upper layer structure (15), and wherein corresponding tongue-and-groove connecting geometries are provided on opposite border sides of the covering element (1).

2. Covering element according to claim 1, **characterized in that** the distance of the functional layer (5) from the at least two side edges (8) is at least 0.1% of the width and/or length of the covering element, preferably between 0.1% to 40%, more preferably between 1% to 20%, and/or **in that** the functional layer (5) is arranged centrally on the main body (4) and/or **in that** the functional layer (5) extends between 1% to 85%, preferably between 5% to 75%, more preferably between 10% to 50% and in particular at least substantially 20%, of the width (9) and/or length (10) of the covering element (1) on the main body (4).

3. Covering element according to claim 1 or 2, **characterized in that** the functional layer (5) is designed as an electrically conductive printed circuit board and/or **in that** the functional layer (5) is arranged on a carrier layer.

4. Covering element according to one of the preceding claims, **characterized in that** the functional layer (5) is designed as an electrically conductive functional layer (5).

5. Covering element according to any one of the preceding claims, **characterized in that** a plurality of functional layers (5) is provided.

6. Covering element according to any one of the preceding claims, **characterized in that** the transmission device (6) comprises at least one antenna for transmitting and/or receiving information.

7. Covering element according to one of the preceding claims, **characterized in that** the transmission device (6) is designed to process the information transmitted by the measuring device (7), in particular wherein the transmission device (6) is designed to encode and/or encrypt the information.

8. Covering element according to one of the preceding claims, **characterized in that** the measuring device (7) has at least one sensor and/or **in that** the measuring device (7) is designed for reading out an access control means (11).

9. Covering element according to any one of the preceding claims, **characterized in that** the functional layer (5) is associated with a processing device (12) for receiving information transmitted by the transmission device (6).

10. Covering element according to one of the preceding claims, **characterized in that** at least one power supply means (14) is associated with the functional layer (5) and/or is integrated into the functional layer (5).

11. System (16) **characterized by** at least one first covering element (1) according to one of the preceding claims, a processing device (12) and at least one further second covering element (2), wherein the first covering element (1) has a main body (4) and at least one functional layer (5), wherein the functional layer (5) has a transmission device (6) for transmitting information and a measuring device (7) for detecting information, wherein the measuring device (7) is coupled to the transmission device (6) in such a way that the transmission device (6) is designed for the wireless transmission of the information received from the measuring device (7), wherein the first covering element (1) is designed to be electrically contactless and electrically contact-free at all side edges (8), wherein the processing device (12) and the first covering element (1) are coupled to one another for the exchange of information, and wherein a plurality of first covering elements (1) is provided, wherein the first covering elements (1) are connected to one another and/or to further second covering elements (2) at the edges by means of tongue-and-groove connections.

12. System according to claim 11, **characterized in that** the processing device (12) is designed to decode the information transmitted and encoded by the transmission device (6) and/or **in that** the processing device (12) is designed to detect the position of the first covering element (1).

13. System according to any one of claims 11 or 12, **characterized in that** a control device (17) and/or an electronic terminal (18) is provided for receiving information transmitted by the processing device (12).

14. System according to any one of claims 11 to 13, **characterized in that** the processing device (12), the measuring device (7), the transmission device (6) and/or the display device (13) are controllable via the control device (17) and/or the electronic terminal (18).

15. System according to any one of claims 11 to 14, **characterized in that** the data of the access control means (11) detected by the measuring device (7) are transmittable to the processing device (12) and/or the control device (17) and/or the electronic terminal (18).

16. System according to any one of claims 11 to 15, **characterized in that** the transmission device (6), the processing device (12), the control device (17) and/or the electronic terminal (18) are coupled to an access control device (19).

17. System according to any one of claims 11 to 16, **characterized in that** the transmission device (6), the processing device (12), the control device (17) and/or the electronic terminal (18) are coupled to an alarm device (20).

## Revendications

1. Élément de revêtement (1) destiné à être utilisé comme revêtement de sol, de mur et/ou de plafond, comprenant un corps principal (4) et au moins une couche fonctionnelle (5), dans lequel la couche fonctionnelle (5) présente un dispositif de transmission (6) pour la transmission d'informations et un dispositif de mesure (7) pour la détection d'informations, le dispositif de mesure (7) étant couplé au dispositif de transmission (6) de telle sorte que le dispositif de transmission (6) est conçu pour la transmission sans fil des informations reçues par le dispositif de mesure (7), l'élément de revêtement (1) étant conçu sans connexion électrique et sans contact électrique sur tout le pourtour de tous les bords latéraux (8), la couche fonctionnelle (5) étant espacée d'au moins deux bords latéraux (8),
**caractérisé en ce que**
la couche fonctionnelle (5) est située sous le corps principal (4), à l'opposé d'une structure de la couche supérieure (15), et dans lequel des géométries de connexion à languette et rainure correspondantes sont prévues sur des côtés de bord opposés de l'élément de revêtement (1).

2. Élément de revêtement selon la revendication 1, **caractérisé en ce que** la distance de la couche fonctionnelle (5) par rapport aux au moins deux bords latéraux (8) est d'au moins 0,1% de la largeur et/ou de la longueur de l'élément de revêtement, de préférence entre 0.1 % à 40 %, de préférence entre 1 % et 20 %, et/ou **en ce que** la couche fonctionnelle (5) est disposée de manière centrale sur le corps principal (4) et/ou **en ce que** la couche fonctionnelle (5) s'étend entre 1 % et 85 %, de préférence entre 5 % et 75 %, de préférence entre 10 % et 50 % et en particulier au moins sensiblement 20 %, de la largeur (9) et/ou de la longueur (10) de l'élément de revêtement (1) sur le corps principal (4).

3. Élément de revêtement selon la revendication 1 ou 2, **caractérisé en ce que** la couche fonctionnelle (5) est conçue comme une carte de circuit imprimé électriquement conductrice et/ou **en ce que** la couche fonctionnelle (5) est disposée sur une couche de support.

4. Élément de revêtement selon l'une des revendications précédentes, **caractérisé en ce que** la couche fonctionnelle (5) est conçue comme une couche fonctionnelle (5) électriquement conductrice.

5. Élément de revêtement selon l'une quelconque des revendications précédentes, **caractérisé en ce qu'**il est prévu une pluralité de couches fonctionnelles (5).

6. Élément de revêtement selon l'une quelconque des revendications précédentes, **caractérisé en ce que** le dispositif de transmission (6) comprend au moins une antenne d'émission et/ou de réception d'informations.

7. Élément de revêtement selon l'une des revendications précédentes, **caractérisé en ce que** le dispositif de transmission (6) est conçu pour traiter les informations transmises par le dispositif de mesure (7), en particulier dans lequel le dispositif de transmission (6) est conçu pour coder et/ou crypter les informations.

8. Élément de revêtement selon l'une des revendications précédentes, **caractérisé en ce que** le dispositif de mesure (7) comporte au moins un capteur et/ou **en ce que** le dispositif de mesure (7) est conçu pour lire un moyen de contrôle d'accès (11).

9. Élément de revêtement selon l'une quelconque des revendications précédentes, **caractérisé en ce que** la couche fonctionnelle (5) est associée à un dispositif de traitement (12) pour recevoir des informations transmises par le dispositif de transmission (6).

10. Élément de revêtement selon l'une des revendications précédentes, **caractérisé en ce qu'**au moins un moyen d'approvisionnement en énergie (14) est associé à la couche fonctionnelle (5) et/ou est intégré à la couche fonctionnelle (5).

11. Système (16) **caractérisé par** au moins un premier élément de revêtement (1) selon l'une des revendications précédentes, un dispositif de traitement (12) et au moins un autre deuxième élément de revêtement (2), le premier élément de revêtement (1) présentant un corps principal (4) et au moins une couche fonctionnelle (5), dans lequel la couche fonctionnelle (5) présente un dispositif de transmission (6) pour la transmission d'informations et un dispositif de mesure (7) pour la détection d'informations, dans lequel le dispositif de mesure (7) est couplé au dispositif de transmission (6) de telle sorte que le dispositif de transmission (6) est conçu pour la transmission sans fil des informations reçues du dispositif de mesure (7), le premier élément de revêtement (1) étant conçu sans connexion électrique et sans contact électrique sur tous les bords latéraux (8), le dispositif de traitement (12) et le premier élément de revêtement (1) étant couplés l'un à l'autre pour l'échange d'informations, et une pluralité de premiers éléments de revêtement (1) étant prévue, les premiers éléments de revêtement (1) étant reliés entre eux et/ou à d'autres seconds éléments de revêtement (2) sur les bords au moyen de liaisons à rainure et languette.

12. Système selon la revendication 11, **caractérisé en ce que** le dispositif de traitement (12) est conçu pour décoder les informations transmises et codées par le dispositif de transmission (6) et/ou **en ce que** le dispositif de traitement (12) est conçu pour détecter la position du premier élément de revêtement (1).

13. Système selon l'une quelconque des revendications 11 ou 12, **caractérisé en ce qu'**un dispositif de commande (17) et/ou un terminal électronique (18) est prévu pour recevoir les informations transmises par le dispositif de traitement (12).

14. Système selon l'une quelconque des revendications 11 à 13, **caractérisé en ce que** le dispositif de traitement (12), le dispositif de mesure (7), le dispositif de transmission (6) et/ou le dispositif d'affichage (13) peuvent être commandés par le dispositif de commande (17) et/ou le terminal électronique (18).

15. Système selon l'une quelconque des revendications 11 à 14, **caractérisé en ce que** les données du moyen de contrôle d'accès (11) détectées par le dispositif de mesure (7) sont transmissibles au dispositif de traitement (12) et/ou au dispositif de commande (17) et/ou au terminal électronique (18).

16. Système selon l'une quelconque des revendications 11 à 15, **caractérisé en ce que** le dispositif de transmission (6), le dispositif de traitement (12), le dispositif de commande (17) et/ou le terminal électronique (18) sont couplés à un dispositif de contrôle d'accès (19).

17. Système selon l'une quelconque des revendications 11 à 16, **caractérisé en ce que** le dispositif de transmission (6), le dispositif de traitement (12), le dispositif de commande (17) et/ou le terminal électronique (18) sont couplés à un dispositif d'alarme (20).
